# EUROPEAN PATENT APPLICATION

(11) **EP 4 692 361 A1**
(43) Date of publication of application: **11.02.2026**
(21) Application number: 24780886.8
(22) Date of filing: 29.03.2024
(51) Int. Cl.: C12P 1/04, C12N 1/21, C12N 15/31, C12N 15/54, C12P 1/02, C12P 1/06, C12P 5/00, C12N 15/52

(54) **METHOD FOR PRODUCING METHYL COMPOUND**

(30) Priority: 31.03.2023 JP 2023056933; 31.03.2023 JP 2023056935; 31.03.2023 JP 2023056939
(71) Applicant: Mitsubishi Chemical Corporation, Tokyo 100-8251 (JP)
(72) Inventor: YUNOMURA, Shuichi, Tokyo 100-8251 (JP)
(74) Representative: Hoffmann Eitle
(86) International application number: PCT/JP2024/013245
(87) International publication number: WO 2024/204792

(57) **Abstract**

An object of the present invention is to provide a novel method that can produce a methyl compound by regenerating SAM and accelerating the methylation reaction of a compound. The present invention provides a method for producing a methyl compound using glycine, serine, or an organic raw material.

## Description

### [Technical Field]

The present invention relates to a method for producing a methyl compound.

### [Background Art]

Various methyltransferases are known in the natural world. These enzymes regioselectively and stereoselectively methylate various compounds as substrates. Most of these methyltransferases transfer a methyl group to target compounds by using S-adenosylmethionine (SAM: S-adenosyl-L-methionine) as a methyl group donor.

SAM serving as a methyl group donor, however, is supplied in a limited amount inside cells and easily depleted.

On the other hand, a method for recycling SAM by adding methanol to a culture medium of a microorganism coexpressing methylase and methanol dehydrogenase is known (Patent Literature 1: International Publication No. WO 2019/160059).

### [Citation List]

### [Patent Literature]

[Patent Literature 1] International Publication No. WO 2019/160059

### [Summary of Invention]

### [Technical Problem]

A method described in Patent Literature 1 involves constructing an S-adenosylmethionine recycling pathway that supplies methyl group carbon through added methanol, but the method did not have a sufficient accelerating effect on the methylation reaction. There is a demand for providing a novel method that can produce a methyl compound by more efficiently regenerating SAM and accelerating the methylation reaction of a compound. Since SAM plays an important role in the methylation of a compound, there is a demand for providing a cell useful in efficient regeneration of SAM.

### [Solution to Problem]

The present inventors have succeeded in producing a methyl compound by using glycine or serine and thereby efficiently regenerating SAM and accelerating methylation reaction, leading to the completion of the present invention. The present inventors have also succeeded in efficiently regenerating SAM from an organic raw material by using a cell engineered so as to enhance the activity or expression of serine hydroxymethyltransferase (GlyA) and S-adenosylmethionine (SAM)-dependent methyltransferase, leading to the completion of the present invention.

Specifically, the present invention is as follows.

[1-1] A method for producing a methyl compound, comprising the step of performing methylation reaction in the presence of glycine or serine using a cell engineered so as to enhance the activity or expression of S-adenosylmethionine-dependent methyltransferase.
[1-2] The method according to [1-1], wherein the cell is a cell further engineered so as to enhance the activity or expression of serine hydroxymethyltransferase (GlyA).
[1-3]
   A method for producing a methyl compound, comprising the step of performing methylation reaction using a cell engineered so as to enhance the activity or expression of serine hydroxymethyltransferase (GlyA) and S-adenosylmethionine-dependent methyltransferase.
[1-4]
   The method according to [1-3], wherein the cell is a cell further engineered so as to enhance the activity or expression of at least one or more enzymes selected from the group consisting of D-3-phosphoglycerate dehydrogenase (SerA), phosphoserine aminotransferase (SerC), and phosphoserine phosphatase (SerB).

[1-5] The method according to any of [1-1] to [1-4], wherein the cell is a cell further engineered so as to enhance the activity or expression of at least one or more enzymes selected from the group consisting of S-adenosylhomocysteine nucleosidase (Mtn), S-ribosylhomocysteine lyase (LuxS), and S-adenosylhomocysteine hydrolase (SahH).
[1-6] The method according to any of [1-1] to [1-5], wherein the cell is a cell further engineered so as to enhance the activity or expression of at least one or more enzymes selected from the group consisting of ribose-phosphate diphosphokinase (Prs), adenine phosphoribosyltransferase (Apt), and adenosine kinase (ADK).
[1-7]
   The method according to any of [1-1] to [1-6], wherein the cell is a cell further engineered so as to enhance the activity or expression of at least one or more enzymes selected from the group consisting of 5,10-methylene-tetrahydrofolate reductase (MetF), methionine synthase (MetH or MetE), and methionine adenosyltransferase (MetK).
[1-8]
   The method according to any of [1-1] to [1-7], wherein the cell is a cell further engineered so as to enhance the activity or expression of at least one or more enzymes selected from the group consisting of aminomethyltransferase (GcvT), octanoyl-[GcvH]:protein N-octanoyltransferase (GcvH), and glycine dehydrogenase (GcvP).

[1-9]
   The method according to any of [1-1] to [1-8], wherein the cell is at least one selected from the group consisting of an animal cell, an insect cell, a plant cell, and a microorganism.
[1-10]
   The method according to [1-9], wherein the cell is a microorganism.
[1-11]
   The method according to [1-10], wherein the microorganism is at least one selected from the group consisting of *E. coli,* a coryneform bacterium, a bacterium of the genus *Bacillus,* a bacterium of the genus *Pantoea,* a bacterium of the genus *Enterobacter,* a bacterium of the genus *Pseudomonas,* a bacterium of the genus *Streptomyces,* a bacterium of the genus *Actinomyces,* a filamentous fungus, and a yeast.

[1-12]
   The method according to any of [1-1] to [1-11], further comprising the step of allowing an organic raw material to act on the cell.
[1-13]
   The method according to [1-12], wherein the organic raw material is an organic compound having 3 or more carbon atoms.
[1-14]
   The method according to [1-12], wherein the organic raw material comprises one selected from the group consisting of a saccharide, an alcohol and an organic acid.
[1-15]
   The method according to [1-1], wherein the cell is a cell cultured in the presence of an organic raw material containing glycine or serine.

[1-16]
   The method according to any of [1-1] to [1-15], further comprising the step of recovering a methyl compound produced through the methylation reaction.
[1-17]
   The method according to any of [1-1] to [1-16], further comprising the step of purifying a methyl compound produced through the methylation reaction.
[1-18]
   The method according to any of [1-12] to [1-14], further comprising the step of recovering a methyl compound produced by allowing the organic raw material to act.
[1-19]
   The method according to any of [1-12] to [1-14] and [1-18], further comprising the step of purifying a methyl compound produced by allowing the organic raw material to act.

[1-20]
   The method according to any of [1-1] to [1-19], wherein the methyl compound is a derivative of an aromatic amino acid.
[1-21]
   A cell engineered so as to enhance the activity or expression of serine hydroxymethyltransferase (GlyA) and S-adenosylmethionine-dependent methyltransferase.
[1-22]
   The cell according to [1-21], further engineered so as to enhance the activity or expression of at least one or more enzymes selected from the group consisting of S-adenosylhomocysteine nucleosidase (Mtn), S-ribosylhomocysteine lyase (LuxS), and S-adenosylhomocysteine hydrolase (SahH).
[1-23]
   The cell according to [1-21] or [1-22], further engineered so as to enhance the activity or expression of at least one or more enzymes selected from the group consisting of ribose-phosphate diphosphokinase (Prs), adenine phosphoribosyltransferase (Apt), and adenosine kinase (ADK).

[1-24]
   The cell according to any of [1-21] to [1-23], further engineered so as to enhance the activity or expression of at least one or more enzymes selected from the group consisting of 5,10-methylene-tetrahydrofolate reductase (MetF), methionine synthase (MetH or MetE), and methionine adenosyltransferase (metK).
[1-25]
   The cell according to any of [1-21] to [1-24], further engineered so as to enhance the activity or expression of at least one or more enzymes selected from the group consisting of aminomethyltransferase (GcvT), octanoyl-[GcvH]:protein N-octanoyltransferase (GcvH), and glycine dehydrogenase (GcvP).
[1-26]
   The cell according to any of [1-21] to [1-25], further engineered so as to enhance the activity or expression of at least one or more enzymes selected from the group consisting of D-3-phosphoglycerate dehydrogenase (SerA), phosphoserine aminotransferase (SerC), and phosphoserine phosphatase (SerB).

[1-27]
   The cell according to any of [1-21] to [1-26], wherein the cell is at least one selected from the group consisting of an animal cell, an insect cell, a plant cell, and a microorganism.
[1-28]
   The cell according to [1-27], wherein the cell is a microorganism.
[1-29]
   The cell according to [1-28], wherein the microorganism is at least one selected from the group consisting of *E. coli,* a coryneform bacterium, a bacterium of the genus *Bacillus, a* bacterium of the genus *Pantoea,* a bacterium of the genus *Enterobacter,* a bacterium of the genus *Pseudomonas,* a bacterium of the genus *Streptomyces,* a bacterium of the genus *Actinomyces,* a filamentous fungus, and a yeast.
[1-30]
   A method for producing an organic compound, comprising the step of producing the organic compound via a methyl compound obtained by the method according to any of [1-1] to [1-20] as an intermediate.

[2-1]
   A method for producing a methyl compound, comprising the step of performing methylation reaction in the presence of glycine using a microorganism engineered so as to enhance the activity or expression of S-adenosylmethionine-dependent methyltransferase.
[2-2]
   The method for producing a methyl compound according to [2-1], wherein the microorganism is a microorganism further engineered so as to enhance the activity or expression of at least one or more enzymes selected from the group consisting of S-adenosylhomocysteine nucleosidase (Mtn), S-ribosylhomocysteine lyase (LuxS), and S-adenosylhomocysteine hydrolase (SahH).
[2-3]
   The method for producing a methyl compound according to [2-1] or [2-2], wherein the microorganism is a microorganism further engineered so as to enhance the activity or expression of at least one or more enzymes selected from the group consisting of 5,10-methylene-tetrahydrofolate reductase (MetF), methionine synthase (MetH or MetE), and methionine adenosyltransferase (MetK).

[2-4]
   The method for producing a methyl compound according to any of [2-1] to [2-3], wherein the microorganism is a microorganism further engineered so as to enhance the activity or expression of at least one or more enzymes selected from the group consisting of aminomethyltransferase (GcvT), octanoyl-[GcvH]:protein N-octanoyltransferase (GcvH), and glycine dehydrogenase (GcvP).
[2-5]
   The method for producing a methyl compound according to any of [2-1] to [2-4], wherein the microorganism is at least one selected from the group consisting of *E. coli, a* coryneform bacterium, a bacterium of the genus *Bacillus,* a bacterium of the genus *Pantoea, a* bacterium of the genus *Enterobacter,* a bacterium of the genus *Pseudomonas,* a bacterium of the genus *Streptomyces,* a bacterium of the genus *Actinomyces,* a filamentous fungus, and a yeast.
[2-6]
   The method for producing a methyl compound according to any of [2-1] to [2-5], wherein the microorganism is a microorganism cultured in the presence of an organic raw material containing glycine.

[2-7]
   The method for producing a methyl compound according to any of [2-1] to [2-6], further comprising the step of recovering a methyl compound produced through the methylation reaction.
[2-8]
   The method for producing a methyl compound according to any of [2-1] to [2-7], further comprising the step of purifying a methyl compound produced through the methylation reaction.
[2-9]
   A method for producing an organic compound, comprising the step of producing the organic compound via a methyl compound obtained by the method according to any of [2-1] to [2-8] as an intermediate.

[3-1]
   A method for producing a methyl compound, comprising the step of performing methylation reaction in the presence of serine using a microorganism engineered so as to enhance the activity or expression of S-adenosylmethionine-dependent methyltransferase.
[3-2]
   The method for producing a methyl compound according to [3-1], wherein the microorganism is a microorganism further engineered so as to enhance the activity or expression of at least one or more enzymes selected from the group consisting of S-adenosylhomocysteine nucleosidase (Mtn), S-ribosylhomocysteine lyase (LuxS), and S-adenosylhomocysteine hydrolase (SahH).
[3-3]
   The method for producing a methyl compound according to [3-1] or [3-2], wherein the microorganism is a microorganism further engineered so as to enhance the activity or expression of at least one or more enzymes selected from the group consisting of 5,10-methylene-tetrahydrofolate reductase (MetF), methionine synthase (MetH or MetE), and methionine adenosyltransferase (MetK).

[3-4]
   The method for producing a methyl compound according to any of [3-1] to [3-3], wherein the microorganism is a microorganism further engineered so as to enhance the activity or expression of serine hydroxymethyltransferase (GlyA).
[3-5]
   The method for producing a methyl compound according to any of [3-1] to [3-4], wherein the microorganism is a microorganism further engineered so as to enhance the activity or expression of at least one or more enzymes selected from the group consisting of aminomethyltransferase (GcvT), octanoyl-[3-GcvH]:protein N-octanoyltransferase (GcvH), and glycine dehydrogenase (GcvP).
[3-6]
   The method for producing a methyl compound according to any of [3-1] to [3-5], wherein the microorganism is at least one selected from the group consisting of *E. coli, a* coryneform bacterium, a bacterium of the genus *Bacillus,* a bacterium of the genus *Pantoea*, a bacterium of the genus *Enterobacter,* a bacterium of the genus *Pseudomonas,* a bacterium of the genus *Streptomyces,* a bacterium of the genus *Actinomyces,* a filamentous fungus, and a yeast.

[3-7]
   The method for producing a methyl compound according to any of [3-1] to [3-6], wherein the microorganism is a microorganism cultured in the presence of an organic raw material containing serine.
[3-8]
   The method for producing a methyl compound according to any of [3-1] to [3-7], further comprising the step of recovering a methyl compound produced through the methylation reaction.
[3-9]
   The method for producing a methyl compound according to any of [3-1] to [3-8], further comprising the step of purifying a methyl compound produced through the methylation reaction.
[3-10]
   A method for producing an organic compound, comprising the step of producing the organic compound via a methyl compound obtained by the method according to any of [3-1] to [3-9] as an intermediate.

[4-1]
   A microorganism engineered so as to enhance the activity or expression of serine hydroxymethyltransferase (GlyA) and S-adenosylmethionine-dependent methyltransferase.
[4-2]
   The microorganism according to [4-1], further engineered so as to enhance the activity or expression of at least one or more enzymes selected from the group consisting of S-adenosylhomocysteine nucleosidase (Mtn), S-ribosylhomocysteine lyase (LuxS), and S-adenosylhomocysteine hydrolase (SahH).
[4-3]
   The microorganism according to [4-1] or [4-2], further engineered so as to enhance the activity or expression of at least one or more enzymes selected from the group consisting of 5,10-methylene-tetrahydrofolate reductase (MetF), methionine synthase (MetH or MetE), and methionine adenosyltransferase (metK).

[4-4]
   The microorganism according to any of [4-1] to [4-3], further engineered so as to enhance the activity or expression of at least one or more enzymes selected from the group consisting of aminomethyltransferase (GcvT), octanoyl-[GcvH]:protein N-octanoyltransferase (GcvH), and glycine dehydrogenase (GcvP).
[4-5]
   The microorganism according to any of [4-1] to [4-4], further engineered so as to enhance the activity or expression of at least one or more enzymes selected from the group consisting of D-3-phosphoglycerate dehydrogenase (SerA), phosphoserine aminotransferase (SerC), and phosphoserine phosphatase (SerB).
[4-6]
   The microorganism according to any of [4-1] to [4-5], wherein the microorganism is at least one selected from the group consisting of *E. coli,* a coryneform bacterium, a bacterium of the genus *Bacillus,* a bacterium of the genus *Pantoea,* a bacterium of the genus *Enterobacter,* a bacterium of the genus *Pseudomonas,* a bacterium of the genus *Streptomyces,* a bacterium of the genus *Actinomyces,* a filamentous fungus, and a yeast.

[4-7]
   A method for producing a methyl compound, comprising the step of allowing an organic raw material to act on a microorganism according to any of [4-1] to [4-6].
[4-8]
   The method for producing a methyl compound according to [4-7], wherein the organic raw material comprises one or more members selected from the group consisting of a saccharide, an alcohol, and an organic acid.

[4-9]
   The method for producing a methyl compound according to [4-7] or [4-8], further comprising the step of recovering a methyl compound produced by allowing the organic raw material to act.
[4-10]
   The method for producing a methyl compound according to any of [4-7] to [4-9], further comprising the step of purifying a methyl compound produced by allowing the organic raw material to act.
[4-11]
   A method for producing an organic compound, comprising the step of producing the organic compound via a methyl compound obtained by the method according to any of [4-7] to [4-10] as an intermediate.

### [Advantageous Effects of Invention]

The present invention can efficiently regenerate SAM and accelerate the methylation reaction of a substance. The present invention can also provide a cell useful in the regeneration of SAM. Furthermore, the present invention can produce a methyl compound. Moreover, the amount of a methyl compound produced can be increased as compared with the case of not using the reaction system of the present invention.

### [Brief Description of Drawings]

[Figure 1] Figure 1 is a diagram showing one example of a reaction system for use in the method of the present invention.
[Figure 2] Figure 2 is a diagram showing one example of a reaction system for use in the method of the present invention.
[Figure 3] Figure 1 is a diagram showing one example of a reaction system for the cell of the present invention.

### [Description of Embodiments]

Hereinafter, the present invention will be described in detail. Embodiments given below are illustrations for describing the present invention, and the present invention is not intended to be limited to only these embodiments. The present invention can be carried out in various forms without departing from the spirit of the present invention.

The present specification encompasses the contents described in the specifications and drawings of Japanese Patent Application Nos. 2023-056933, 2023-056935, and 2023-056939 filed on March 31, 2023, based on which the priority of the present application is claimed.

### 1. Summary

SAM is useful in the methylation reaction of a compound, but inside cells, it is supplied in a limited amount and easily depleted.

A method for recycling SAM by adding methanol to a culture medium of a microorganism coexpressing methylase and methanol dehydrogenase has heretofore been known.

However, no method for efficiently producing a methyl compound by using glycine or serine and accelerating methylation reaction has been known.

Against this background, the present inventor has succeeded in using glycine or serine and thereby efficiently regenerating SAM and accelerating methylation reaction, leading to the completion of the present invention.

Since SAM plays an important role in the methylation of a compound, there is a demand for a cell useful in efficient regeneration of SAM. The present inventor has succeeded in producing a methyl compound by using a cell engineered so as to enhance the activity or expression of serine hydroxymethyltransferase (GlyA) and S-adenosylmethionine (SAM)-dependent methyltransferase and thereby efficiently regenerating SAM from an organic raw material and accelerating methylation reaction, leading to the completion of the present invention.

### 2-1. Method for producing methyl compound using glycine

### (1) Glycine

Glycine, an amino acid, is cleaved by a glycine decarboxylase complex constituted by aminomethyltransferase (GcvT), octanoyl-[GcvH]:protein N-octanoyltransferase (GcvH), glycine dehydrogenase (GcvP), and dihydrolipoyl dehydrogenase (LpdA). Through this reaction, tetrahydrofolate is converted to 5,10-methylenetetrahydrofolate (glycine cleavage system).

### (2) S-Adenosylmethionine

S-Adenosylmethionine (SAM; S-adenosyl-L-methionine) is a substance that functions as a methyl group donor when methyltransferase transfers a methyl group to a methylation target substance (substance to be methylated). SAM becomes S-adenosyl-L-homocysteine (SAH) when losing its methyl group (demethylated) by transferring the methyl group to a methylation target substance. SAH is degraded into homocysteine (Hcy) through S-ribosylhomocysteine (SRH). Since SAM is supplied in a limited amount inside cells, SAM is easily depleted unless methionine or SAM is newly supplied.

### (3) S-Adenosylmethionine-dependent methyltransferase

S-Adenosylmethionine (SAM)-dependent methyltransferase is an enzyme that transfers a methyl group of SAM to a methylation target substance (substance to be methylated). Examples of the methylation target substance include DNAs, proteins, and low-molecular compounds that are subject to methylation. A low-molecular compound is preferred. SAM is demethylated by S-adenosylmethionine-dependent methyltransferase and thereby converted to SAH. On the other hand, the methylation target substance is methylated to thereby produce a methyl compound. In the present specification, the S-adenosylmethionine-dependent methyltransferase is also simply referred to as "methyltransferase."

In the present invention, the methyltransferase is not limited as long as the methyltransferase transfers a methyl group of SAM to a methylation target substance. Examples of such methyltransferase include, but are not limited to, methyltransferase that methylates a DNA, methyltransferase that methylates a protein, and a transferase that methylates a low-molecular compound. Alternatively, the methyltransferase can be obtained, for example, by searching a known database BRENDA (https://www.brenda-enzyme) for "methyltransferase" as a keyword and selecting an arbitrary enzyme described as "S-adenosyl-L-methionine" in the section "Synonyms." Examples of such methyltransferase include, but are not limited to, L-tyrosine C3-methyltransferase, L-histidine N-alpha-methyltransferase, perillyl acid O-methyltransferase, and catechol O-methyltransferase.

### (4) Cell

In the present invention, a cell engineered so as to enhance the activity or expression of S-adenosylmethionine (SAM)-dependent methyltransferase can be used. The cell used in the present invention is not limited as long as SAM exists in the cell. Examples thereof include microorganisms, animal (mammalian) cells, insect cells, and plant cells (including cultured plant cells). Examples of the microorganism include, but are not limited to, *E. coli,* coryneform bacteria, bacteria of the genus *Bacillus,* bacteria of the genus *Pantoea,* bacteria of the genus *Enterobacter,* bacteria of the genus *Pseudomonas,* bacteria of the genus *Streptomyces,* bacteria of the genus *Actinomyces,* filamentous fungi, and yeasts. Examples of the animal cell include, but are not limited to, mouse cells, rat cells, rabbit cells, dog cells, monkey cells, and human-derived cultured cells. The insect cell is not limited as long as the cell permits protein expression by baculovirus or the like. Examples of the plant cell include, but are not limited to, cells of plants of the genus *Nicotiana* (e.g., *Nicotiana benthamiana*, *N. tabacum*, and *N. excelsior*), plants of the family *Solanaceae*, plants of the family *Poaceae,* plants of the family *Brassicaceae*, plants of the family *Compositae*, and bryophytes. The cell used in the present invention is preferably a cell capable of expressing a foreign gene.

A cell (e.g., a microorganism) engineered so as to enhance the activity or expression of S-adenosylmethionine (SAM)-dependent methyltransferase can be used as the cell used in the present invention. As used herein, the "cell engineered" includes a "microorganism engineered."

In the present invention, a cell engineered so as to enhance the activity or expression of at least one or more enzymes selected from the group consisting of S-adenosylhomocysteine nucleosidase (Mtn), S-ribosylhomocysteine lyase (LuxS), and S-adenosylhomocysteine hydrolase (SahH) (e.g., only SahH, or Mtn and LuxS) in addition to SAM-dependent methyltransferase can also be used.

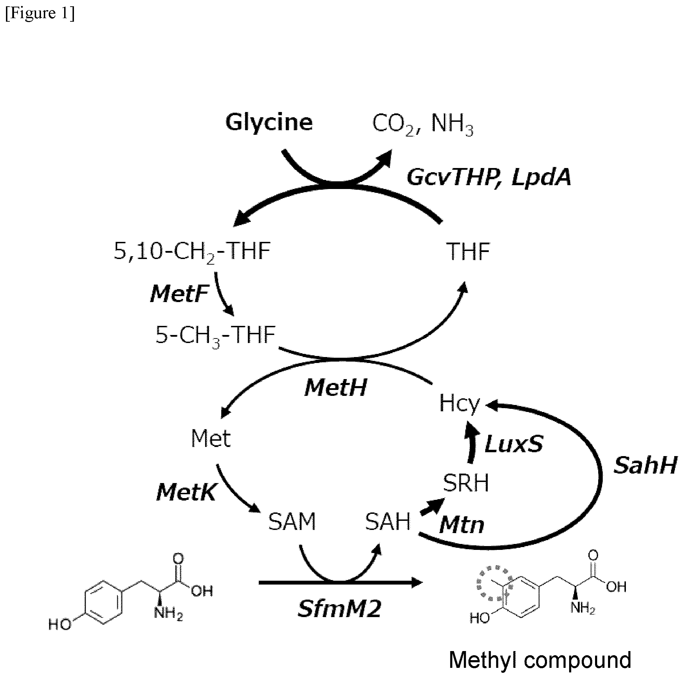

Mtn is an enzyme that is utilized in the conversion of S-adenosylhomocysteine (SAH) to S-ribosylhomocysteine (SRH); LuxS is an enzyme that is utilized in the conversion of SRH to homocysteine (Hcy); and SahH is an enzyme that is utilized in direct conversion of SAH to Hcy (without the conversion to SRH) (Figure 1).

In the present invention, a cell engineered so as to enhance the activity or expression of
(i) at least one or more enzymes selected from the group consisting of Mtn, LuxS, and SahH (e.g., only SahH, or Mtn and LuxS), and/or
(ii) at least one or more enzymes selected from the group consisting of ribose-phosphate diphosphokinase (Prs), adenine phosphoribosyltransferase (Apt), and adenosine kinase (ADK) (e.g., Prs and Apt or only ADK)
in addition to SAM-dependent methyltransferase can be further used.

In the present invention, a cell engineered so as to enhance the activity or expression of the following enzymes (i) to (iii):
(i) at least one or more enzymes selected from the group consisting of Mtn, LuxS, and SahH (e.g., only SahH, or Mtn and LuxS),
(ii) at least one or more enzymes selected from the group consisting of ribose-phosphate diphosphokinase (Prs), adenine phosphoribosyltransferase (Apt), and adenosine kinase (ADK) (e.g., Prs and Apt or only ADK), and
(iii) at least one or more enzymes selected from the group consisting of 5,10-methylene-tetrahydrofolate reductase (MetF), methionine synthase (MetH or MetE), and methionine adenosyltransferase (MetK)
in addition to SAM-dependent methyltransferase can be further used.

The methionine synthase may be any of a cobalamin-dependent form (MetH) and a cobalamin-independent form (MetE).

MetF is an enzyme that is utilized in the production of 5-methyltetrahydrofolate (5-CH₃-THF) from 5,10-methylene-tetrahydrofolate (5,10-CH₂-THF). Methionine synthase (MetH or MetE) is an enzyme that is utilized in reaction through which a methyl group of 5-methyltetrahydrofolate (5-CH₃-THF) is transferred to Hcy so that Hcy is converted to methionine. MetK is an enzyme that is utilized in the conversion of methionine to SAM (Figure 1).

The conversion of methionine to SAM requires adenosine triphosphate (ATP). This ATP can be synthesized through the reaction of at least one or more enzymes selected from the group consisting of ribose-phosphate diphosphokinase (Prs), adenine phosphoribosyltransferase (Apt), and adenosine kinase (ADK).

Prs can synthesize phosphoribosyl pyrophosphate (PRPP) from ribose-5-phosphate (R5P), a metabolite of the pentose phosphate pathway. Apt can synthesize adenosine monophosphate (AMP) from PRPP and adenine. ADK can synthesize AMP. AMP can be further phosphorylated and thereby converted to ATP.

Adenine is a metabolite resulting from the reaction of Mtn, and adenosine is a metabolite resulting from the reaction of SahH. These metabolites are utilized as substrates for the synthesis of ATP so that ATP necessary for the synthesis of SAM can be efficiently supplied.

In the present invention, a cell engineered so as to enhance the activity or expression of at least one or more enzymes selected from the group consisting of the following enzymes (i) to (iv):
(i) at least one or more enzymes selected from the group consisting of Mtn, LuxS, and SahH (e.g., only SahH, or Mtn and LuxS),
(ii) at least one or more enzymes selected from the group consisting of ribose-phosphate diphosphokinase (Prs), adenine phosphoribosyltransferase (Apt), and adenosine kinase (ADK) (e.g., Prs and Apt or only ADK), and
(iii) at least one or more enzymes selected from the group consisting of MetF, methionine synthase (MetH or MetE), and MetK, and
(iv) at least one or more enzymes selected from the group consisting of aminomethyltransferase (GcvT), octanoyl-[GcvH]:protein N-octanoyltransferase (GcvH), and glycine dehydrogenase (GcvP)
in addition to SAM-dependent methyltransferase can be further used.

GcvT, GcvH, and GcvP are enzymes constituting a glycine decarboxylase complex, and this complex cleaves glycine. Through this reaction, tetrahydrofolate (THF) is converted to 5,10-methylene-tetrahydrofolate (5,10-CH₂-THF) (glycine cleavage system) (Figure 1).

Examples of the cell engineered so as to enhance the activity or expression of SAM-dependent methyltransferase include cells comprising a DNA encoding SAM-dependent methyltransferase. The SAM-dependent methyltransferase can be appropriately selected on the basis of a known database, for example, BRENDA (https://www.brenda-enzyme), as described above.

Examples of the cell engineered so as to enhance the activity or expression of each of Mtn, LuxS, SahH, Prs, Apt, ADK, MetF, MetH, MetE, MetK, GcvT, GcvH, and GcvP include cells comprising a DNA encoding each of Mtn, LuxS, SahH, Prs, Apt, ADK, MetF, MetH, MetE, MetK, GcvT, GcvH, and GcvP.

In the present invention, the "cell engineered" and the cell comprising a DNA encoding each enzyme can also be referred to as a "transformant."

In the present invention, the "microorganism engineered" and the microorganism comprising a DNA encoding each enzyme can also be referred to as a "transformant."

The DNA encoding each of SAM-dependent methyltransferase, Mtn, LuxS, SahH, Prs, Apt, ADK, MetF, MetH, MetE, MetK, GcvT, GcvH, and GcvP can be prepared by obtaining information on the nucleotide sequence thereof from a known database, for example, NCBI (https://www.ncbi.nlm.nih.gov/nucleotide/) and using a known genetic engineering approach.

In the present invention, a vector comprising the DNA encoding each enzyme can be used for transfecting a cell with the DNA. Examples of such a vector include plasmid vectors and virus vectors. Those skilled in the art can appropriately select the type of the vector depending on the type of a host cell (e.g., a host microorganism) and a purpose. The vector of the present invention can comprise, in addition to the DNA encoding the enzyme, cis elements such as a promoter and an enhancer, a splicing signal, a poly-A addition signal, a ribosomal binding sequence (SD sequence), a selective marker gene, a reporter gene, and the like, and these elements may be engineered.

The vector of the present invention is not limited as long as the enzyme used in the present invention can be expressed by a host cell. For example, a vector that enables two or more enzyme genes to be expressed can be used. Examples of such a vector, in the case of a plasmid vector, include, but are not limited to, pET Duet-1, pCOLA Duet-1, pACYC Duet-1, and pCDF Duet-1 (Novagen).

In the present invention, the vector can comprise DNAs encoding one or more enzymes. For example, one vector may comprise DNAs encoding Mtn and LuxS, DNAs encoding GcvT, GcvH, and GcvP, DNAs encoding MetF, MetH, and MetK, DNAs encoding MetF, MetH, MetK, Mtn, and Lux S, DNAs encoding Prs and Apt, DNAs encoding Prs, Apt, and ADK, or a DNA encoding ADK.

In the present invention, for example, a glycine cleavage reaction system can be introduced to a cell by introducing DNAs encoding GcvT, GcvH, and GcvP to the cell. In the present invention, for example, a glycine cleavage reaction system may be introduced to a cell by introducing DNAs encoding GcvT, GcvH, and GcvP, as well as LpdA, to the cell. Specifically, in the present invention, a cell having a glycine cleavage reaction system can be used.

The microorganism used in the present invention is not limited as long as the microorganism can express SAM-dependent methyltransferase. Examples thereof include *E*. *coli*, coryneform bacteria, bacteria of the genus *Bacillus,* bacteria of the genus *Pantoea,* bacteria of the genus *Enterobacter,* bacteria of the genus *Pseudomonas,* bacteria of the genus *Streptomyces,* bacteria of the genus *Actinomyces,* filamentous fungi, and yeasts.

Those skilled in the art can appropriately set the culture conditions (a temperature, a time, composition of a medium, etc.) of the cell used in the present invention and the reaction conditions (a temperature, a reaction time, pH, etc.) of the enzyme used in the present invention depending on the type of the cell.

The cell used in the present invention is preferably a cell cultured in the presence of an organic raw material containing glycine, i.e., a cell cultured in the presence of an organic raw material containing glycine before the start of methylation reaction (before addition of a methylation target substance). The present inventor has found the cell engineered according to the present invention can be cultured in the presence of glycine to thereby accelerate methylation reaction.

As used herein, the "organic raw material" means a carbon source or a nitrogen source that can be assimilated by a cell (e.g., a microorganism) for proliferation. Examples of the carbon source include, but are not limited to, organic compounds having 3 or more carbon atoms, for example, saccharides, alcohols and organic acids. Examples of the nitrogen source include, but are not limited to, organic nitrogen compounds. In the present invention, examples of the "saccharide" include, but are not limited to, glucose, fructose, sucrose, xylose, arabinose, and cellulose. In the present invention, examples of the "alcohol" include, but are not limited to, methanol, ethanol, propanol, butanol, glycerol, mannitol, and inositol. In the present invention, examples of the "organic acid" include, but are not limited to, formic acid, acetic acid, benzoic acid, citric acid, lactic acid, oxalic acid, and succinic acid. In the present invention, examples of the "organic nitrogen compound" include, but are not limited to, urea, soybean hydrolysates, casein degradants, peptone, yeast extracts, meat extracts, and corn steep liquor.

### (5) Methyl compound

As used herein, the "methyl compound" means a compound having a methyl group. Specifically, the methyl compound according to the present invention is, for example, a compound having a methyl group derived from SAM (transferred from SAM). In the present invention, the methyl compound results from, for example, the transfer of a methyl group of SAM to a methylation target substance by the action of SAM-dependent methyltransferase. Specifically, the methyl compound according to the present invention is not limited as long as the methyl compound has been produced or can be produced in the presence of SAM. Examples of the methyl compound according to the present invention include derivatives of aromatic amino acids. Examples of the aromatic amino acid include tyrosine, histidine, tryptophan, and phenylalanine. Examples of the methyl compound which is a derivative of an aromatic amino acid include, but are not limited to, 3-methyltyrosine, hercynine, ergothioneine, melatonin, vanillic acid, vanillin, isovanillic acid, ferulic acid, isoferulic acid, pinostilbene, pterostilbene, reticuline, thebaine, codeine, magnoflorine, berberine, ononitol, and pinitol.

In one aspect of the present invention, the methyl compound may be at least one selected from the group consisting of, for example, 3-methyltyrosine, hercynine, ergothioneine, melatonin, reticuline, vanillic acid, vanillin, isovanillic acid, ferulic acid, isoferulic acid, pinostilbene, pterostilbene, thebaine, codeine, magnoflorine, berberine, ononitol, and pinitol.

In an alternative aspect of the present invention, the methyl compound may be at least one selected from the group consisting of, for example, 3-methyltyrosine, hercynine, ergothioneine, melatonin, and reticuline.

In an alternative aspect of the present invention, the methyl compound may be at least one selected from the group consisting of, for example, 3-methyltyrosine, hercynine, ergothioneine, and melatonin.

### (6) Method for producing methyl compound

In the present invention, the method for producing a methyl compound comprises the step of performing methylation reaction in the presence of glycine using a cell engineered so as to enhance the activity or expression of S-adenosylmethionine-dependent methyltransferase.

In the present invention, as described above, a cell engineered so as to enhance the activity or expression of at least one or more enzymes selected from the group consisting of the following enzymes (i) to (iv):
(i) at least one or more enzymes selected from the group consisting of Mtn, LuxS, and SahH (e.g., only SahH, or Mtn and LuxS),
(ii) at least one or more enzymes selected from the group consisting of ribose-phosphate diphosphokinase (Prs), adenine phosphoribosyltransferase (Apt), and adenosine kinase (ADK) (e.g., Prs and Apt or only ADK),
(iii) at least one or more enzymes selected from the group consisting of MetF, methionine synthase (MetH or MetE), and MetK, and
(iv) at least one or more enzymes selected from the group consisting of aminomethyltransferase (GcvT), octanoyl-[GcvH]:protein N-octanoyltransferase (GcvH), and glycine dehydrogenase (GcvP)
in addition to SAM-dependent methyltransferase can also be used as the cell.

The method for producing a methyl compound according to the present invention may comprise, for example, the steps of:
(a) preparing and culturing a cell (e.g., a microorganism) comprising at least one DNA selected from (i) a DNA encoding SAM-dependent methyltransferase, (ii) DNAs encoding at least one or more enzymes selected from the group consisting of Mtn, LuxS, and SahH, (ii) DNAs encoding at least one or more enzymes selected from the group consisting of ribose-phosphate diphosphokinase (Prs), adenine phosphoribosyltransferase (Apt), and adenosine kinase (ADK), (iii) DNAs encoding at least one or more enzymes selected from the group consisting of MetF, methionine synthase (MetH or MetE), and MetK, and (iv) DNAs encoding at least one or more enzymes selected from the group consisting of GcvT, GcvH, and GcvP; and
(b) adding glycine to a culture solution of the cell obtained in step (a), followed by further culture.

In step (a), the culture can be performed in the presence of glycine.

The production method of the present invention can further comprise the step of recovering a methyl compound produced through the methylation reaction.

The production method of the present invention can further comprise the step of purifying a methyl compound produced through the methylation reaction.

Those skilled in the art can recover and purify the produced methyl compound by a known method suitable for the physical properties of the methyl compound, for example, treatment such as distillation, membrane dehydration, salting-out using an ion-exchange resin or the like, crystallization, or column chromatography.

In the present invention, the method for producing a methyl compound, in the case of using *E. coli* as a microorganism, can be performed, for example, as follows, although the method is not limited thereto.

First, the host *E. coli* is transfected with a DNA encoding each enzyme to prepare a transformant. The transformant is cultured overnight at approximately 37°C in a known culture medium (e.g., LB medium). Next, the transformant that has proliferated is suspended in a minimum medium (e.g., M9 medium), cultured at approximately 30°C for an appropriate time (e.g., 6 hours), then induced to express the gene using IPTG or the like, and further cultured for an appropriate time (e.g., 16 hours). A transformant with enhanced activity or expression of each enzyme can thereby be prepared. The minimum medium can comprise glycine.

Next, the transformant with enhanced activity or expression of each enzyme and a glycine-containing solution (reaction buffer) are contacted with each other (e.g., the transformant is suspended in a glycine-containing solution). A methylation target substance is added thereto, and the transformant is further cultured at an appropriate temperature (e.g., approximately 30°C) for an appropriate time (e.g., approximately 6 to 24 hours) so that methylation reaction occurs to produce a methyl compound.

The glycine-containing solution can comprise, in addition to glycine, for example, a buffer solution, and glucose as a source of ATP supply, and may further comprise a divalent metal salt (e.g., magnesium sulfate).

### 2-2. Method for producing methyl compound using serine

### (1) Serine

Serine, an amino acid, is converted to glycine by serine hydroxymethyltransferase (GlyA). Through this reaction, tetrahydrofolate is converted to 5,10-methylenetetrahydrofolate.

### (2) S-Adenosylmethionine

S-Adenosylmethionine (SAM; S-adenosyl-L-methionine) is as described in the above section "2-1(2)."

### (3) S-Adenosylmethionine-dependent methyltransferase

S-Adenosylmethionine (SAM)-dependent methyltransferase is as described in the above section "2-1(3)."

### (4) Cell

The type of the "cell" that can be used in the present invention is as described in the above section "2-1(4)." Specifically, examples of the "cell" according to the present invention include, but are not limited to, microorganisms, animal cells, insect cells, and plant cells (including cultured plant cells).

A cell (e.g., a microorganism) engineered so as to enhance the activity or expression of S-adenosylmethionine (SAM)-dependent methyltransferase can be used as the cell used in the present invention.

In the present invention, a cell engineered so as to enhance the activity or expression of at least one or more enzymes selected from the group consisting of S-adenosylhomocysteine nucleosidase (Mtn), S-ribosylhomocysteine lyase (LuxS), and S-adenosylhomocysteine hydrolase (SahH) (e.g., only SahH, or Mtn and LuxS) in addition to SAM-dependent methyltransferase can also be used.

In the present invention, a cell engineered so as to enhance the activity or expression of
(i) at least one or more enzymes selected from the group consisting of Mtn, LuxS, and SahH (e.g., only SahH, or Mtn and LuxS), and/or
(ii) at least one or more enzymes selected from the group consisting of ribose-phosphate diphosphokinase (Prs), adenine phosphoribosyltransferase (Apt), and adenosine kinase (ADK) (e.g., Prs and Apt or only ADK)
in addition to SAM-dependent methyltransferase can be further used.

In the present invention, a cell engineered so as to enhance the activity or expression of the following enzymes (i) to (iii):
(i) at least one or more enzymes selected from the group consisting of Mtn, LuxS, and SahH (e.g., only SahH, or Mtn and LuxS),
(ii) at least one or more enzymes selected from the group consisting of ribose-phosphate diphosphokinase (Prs), adenine phosphoribosyltransferase (Apt), and adenosine kinase (ADK) (e.g., Prs and Apt or only ADK), and
(iii) at least one or more enzymes selected from the group consisting of 5,10-methylene-tetrahydrofolate reductase (MetF), methionine synthase (MetH or MetE), and methionine adenosyltransferase (MetK)
in addition to SAM-dependent methyltransferase can be further used.

The methionine synthase may be any of a cobalamin-dependent form (MetH) and a cobalamin-independent form (MetE).

In the present invention, a cell engineered so as to enhance the activity or expression of at least one or more enzymes selected from the group consisting of the following enzymes (i) to (iv):
(i) at least one or more enzymes selected from the group consisting of Mtn, LuxS, and SahH (e.g., only SahH, or Mtn and LuxS),
(ii) at least one or more enzymes selected from the group consisting of ribose-phosphate diphosphokinase (Prs), adenine phosphoribosyltransferase (Apt), and adenosine kinase (ADK) (e.g., Prs and Apt or only ADK), and
(iii) at least one or more enzymes selected from the group consisting of MetF, methionine synthase (MetH or MetE), and MetK, and
(iv) at least one or more enzymes selected from the group consisting of aminomethyltransferase (GcvT), octanoyl-[GcvH]:protein N-octanoyltransferase (GcvH), and glycine dehydrogenase (GcvP)
in addition to SAM-dependent methyltransferase can be further used.

In the present invention, a cell engineered so as to enhance the activity or expression of at least one or more enzymes selected from the group consisting of the following enzymes (i) to (v):
(i) at least one or more enzymes selected from the group consisting of Mtn, LuxS, and SahH (e.g., only SahH, or Mtn and LuxS),
(ii) at least one or more enzymes selected from the group consisting of ribose-phosphate diphosphokinase (Prs), adenine phosphoribosyltransferase (Apt), and adenosine kinase (ADK) (e.g., Prs and Apt or only ADK), and
(iii) at least one or more enzymes selected from the group consisting of MetF, methionine synthase (MetH or MetE), and MetK,
(iv) at least one or more enzymes selected from the group consisting of aminomethyltransferase (GcvT), octanoyl-[GcvH]:protein N-octanoyltransferase (GcvH), and glycine dehydrogenase (GcvP), and
(v) serine hydroxymethyltransferase (GlyA)
in addition to SAM-dependent methyltransferase can be further used.

Mtn, LuxS, SahH, Prs, Apt, ADK, MetF, methionine synthase (MetH or MetE), MetK, GcvT, GcvH, and GcvP are as described in the above section "2-1(4)."

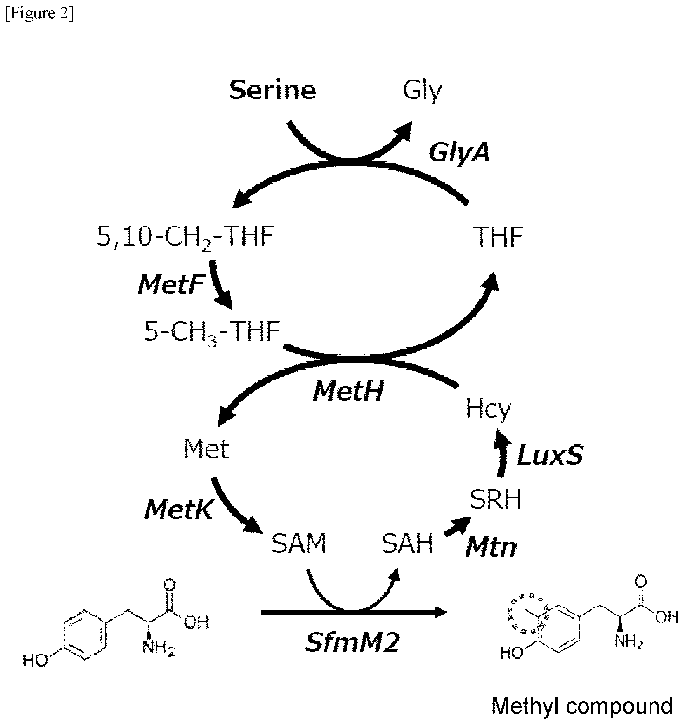

Serine hydroxymethyltransferase (GlyA) is an enzyme that is utilized in the conversion of serine to glycine. Through this reaction, tetrahydrofolate (THF) is converted to 5,10-methylenetetrahydrofolate (5-CH₃-THF) (Figure 2).

Examples of the cell engineered so as to enhance the activity or expression of SAM-dependent methyltransferase include cells comprising a DNA encoding SAM-dependent methyltransferase. The SAM-dependent methyltransferase can be appropriately selected on the basis of a known database, for example, BRENDA (https://www.brenda-enzyme), as described above.

Examples of the cell engineered so as to enhance the activity or expression of each of Mtn, LuxS, SahH, Prs, Apt, ADK, MetF, MetH, MetE, MetK, GcvT, GcvH, GcvP, and GlyA include cells comprising a DNA encoding each of Mtn, LuxS, SahH, Prs, Apt, ADK, MetF, MetH, MetE, MetK, GcvT, GcvH, GcvP, and GlyA.

In the present invention, the "cell engineered" and the cell comprising a DNA encoding each enzyme can also be referred to as a "transformant."

In the present invention, the "microorganism engineered" and the microorganism comprising a DNA encoding each enzyme can also be referred to as a "transformant."

The DNA encoding each of SAM-dependent methyltransferase, Mtn, LuxS, SahH, Prs, Apt, ADK, MetF, MetH, MetE, MetK, GcvT, GcvH, GcvP, and GlyA can be prepared by obtaining information on the nucleotide sequence thereof from a known database, for example, NCBI (https://www.ncbi.nlm.nih.gov/nucleotide/) and using a known genetic engineering approach.

In the present invention, a vector comprising the DNA encoding each enzyme can be used for transfecting a cell with the DNA. The type of the vector that can be used in the present invention is as described in the above section "2-1(4)."

In the present invention, the vector can comprise DNAs encoding one or more enzymes. For example, one vector may comprise DNAs encoding MetF, MetH or MetE, MetK, Mtn, and LuxS, DNAs encoding MetF, MetH or MetE, and MetK, DNAs encoding Mtn and LuxS, DNAs encoding GcvT, GcvH, and GcvP, DNAs encoding Prs and Apt, DNAs encoding Prs, Apt, and ADK, or a DNA encoding ADK.

The culture conditions of the cell used in the present invention are as described in the above section "2-1(4)."

The cell used in the present invention is preferably a cell cultured in the presence of an organic raw material containing serine, i.e., a cell cultured in the presence of an organic raw material containing serine before the start of methylation reaction (before addition of a methylation target substance). The present inventor has found the cell engineered according to the present invention can be cultured in the presence of serine to thereby accelerate methylation reaction.

The "organic raw material" used in the present invention is as described in the above section "2-1(4)."

### (5) Methyl compound

The "methyl compound" according to the present invention is as described in the above section "2-1(5)."

### (6) Method for producing methyl compound

In the present invention, the method for producing a methyl compound comprises the step of performing methylation reaction in the presence of serine using a cell engineered so as to enhance the activity or expression of S-adenosylmethionine-dependent methyltransferase.

In the present invention, as described above, a cell engineered so as to enhance the activity or expression of at least one or more enzymes selected from the group consisting of the following enzymes (i) to (v):
(i) at least one or more enzymes selected from the group consisting of Mtn, LuxS, and SahH (e.g., only SahH, or Mtn and LuxS),
(ii) at least one or more enzymes selected from the group consisting of ribose-phosphate diphosphokinase (Prs), adenine phosphoribosyltransferase (Apt), and adenosine kinase (ADK) (e.g., Prs and Apt or only ADK),
(iii) at least one or more enzymes selected from the group consisting of MetF, methionine synthase (MetH or MetE), and MetK,
(iv) at least one or more enzymes selected from the group consisting of aminomethyltransferase (GcvT), octanoyl-[GcvH]:protein N-octanoyltransferase (GcvH), and glycine dehydrogenase (GcvP), and
(v) serine hydroxymethyltransferase (GlyA)
in addition to SAM-dependent methyltransferase can also be used as the cell.

The method for producing a methyl compound according to the present invention may comprise, for example, the steps of:
(a) preparing and culturing a cell comprising at least one DNA selected from (i) a DNA encoding SAM-dependent methyltransferase, (ii) DNAs encoding at least one or more enzymes selected from the group consisting of Mtn, LuxS, and SahH, (ii) DNAs encoding at least one or more enzymes selected from the group consisting of ribose-phosphate diphosphokinase (Prs), adenine phosphoribosyltransferase (Apt), and adenosine kinase (ADK), (iii) DNAs encoding at least one or more enzymes selected from the group consisting of MetF, methionine synthase (MetH or MetE), and MetK, (iv) DNAs encoding at least one or more enzymes selected from the group consisting of GcvT, GcvH, and GcvP, and (v) a DNA encoding serine hydroxymethyltransferase (GlyA); and
(b) adding serine to a culture solution of the cell obtained in step (a), followed by further culture.

In step (a), the culture can be performed in the presence of serine.

The recovery and purification of a methyl compound in the production method of the present invention are as described in the above section "2-1(6)."

In the present invention, the method for producing a methyl compound using serine, in the case of using *E. coli* as a microorganism, is the same as the method using glycine described in the above section "2-1(6)."

### 2-3. Cell having ability to regenerate S-adenosylmethionine

### (1) S-Adenosylmethionine

S-Adenosylmethionine (SAM; S-adenosyl-L-methionine) is as described in the above section "2-1(2)."

### (2) Regeneration of S-adenosylmethionine

As used herein, the "regeneration" of SAM means that SAM is produced without adding methionine or SAM from the outside of the system. Those skilled in the art can evaluate whether or not SAM has been produced by use of a known method, for example, a method of measuring methyltransferase activity in a test sample. For example, SAM can be evaluated by measuring methyltransferase activity in a test sample and determining that it has been produced when the activity in the test sample is higher than that of a control. A sample differing in one condition from the test sample, for example, a sample that is not supplemented with a substance to be added to the test sample, can be used as the control. The methyltransferase activity can be evaluated, for example, by introducing foreign methyltransferase gene to a cell (e.g., a microorganism), inducing the expression of the enzyme, then adding a methylation target substance to a cell culture solution so as to react with the enzyme, and measuring the amount of a methyl compound produced by mass spectrometry such as LC-MS. Specifically, when the methyltransferase activity in the test sample is higher than that of a control without adding methionine or SAM from the outside of the reaction system, it can be concluded that SAM has been regenerated in this test sample. However, in the present invention, the method for measuring methyltransferase activity is not limited to the method described above, and those skilled in the art can appropriately adopt a known method.

The phrase "higher than that of a control" means that the methyltransferase activity is higher by 5% or more, 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, 95% or more, or 100% or more than that of a control.

### (3) Cell

The type of the "cell" that can be used in the present invention is as described in the above section "2-1(4)." Specifically, examples of the "cell" according to the present invention include, but are not limited to, microorganisms, animal cells, insect cells, and plant cells (including cultured plant cells).

As used herein, the cell having the ability to regenerate S-adenosylmethionine means a cell (e.g., a microorganism) engineered so as to efficiently regenerate SAM.

In the present invention, for example, a cell engineered so as to enhance the activity or expression of serine hydroxymethyltransferase (GlyA) and S-adenosylmethionine (SAM)-dependent methyltransferase can be used as such a cell.

In the present invention, serine hydroxymethyltransferase (GlyA) is an enzyme that converts serine to glycine and also converts tetrahydrofolate to 5,10-methylenetetrahydrofolate.

In the present invention, S-adenosylmethionine (SAM)-dependent methyltransferase is an enzyme that transfers a methyl group of SAM to a methylation target substance (substance to be methylated). The S-adenosylmethionine (SAM)-dependent methyltransferase is as described in the above section "2-1(3)."

In the present invention, a cell engineered so as to enhance the activity or expression of at least one or more enzymes selected from the group consisting of S-adenosylhomocysteine nucleosidase (Mtn), S-ribosylhomocysteine lyase (LuxS), and S-adenosylhomocysteine hydrolase (SahH) (e.g., only SahH, or Mtn and LuxS) in addition to GlyA and SAM-dependent methyltransferase can be used.

In the present invention, a cell engineered so as to enhance the activity or expression of
(i) at least one or more enzymes selected from the group consisting of Mtn, LuxS, and SahH (e.g., only SahH, or Mtn and LuxS), and/or
(ii) at least one or more enzymes selected from the group consisting of ribose-phosphate diphosphokinase (Prs), adenine phosphoribosyltransferase (Apt), and adenosine kinase (ADK) (e.g., Prs and Apt or only ADK)
in addition to GlyA and SAM-dependent methyltransferase can be further used.

In the present invention, a cell engineered so as to enhance the activity or expression of the following enzymes (i) to (iii):
(i) at least one or more enzymes selected from the group consisting of Mtn, LuxS, and SahH (e.g., only SahH, or Mtn and LuxS),
(ii) at least one or more enzymes selected from the group consisting of ribose-phosphate diphosphokinase (Prs), adenine phosphoribosyltransferase (Apt), and adenosine kinase (ADK) (e.g., Prs and Apt), and
(iii) at least one enzyme selected from the group consisting of 5, 10-methylene-tetrahydrofolate reductase (MetF), methionine synthase (MetH or MetE), and methionine adenosyltransferase (metK)
in addition to GlyA and SAM-dependent methyltransferase can be further used.

The methionine synthase may be any of a cobalamin-dependent form (MetH) and a cobalamin-independent form (MetE).

In the present invention, a cell engineered so as to enhance the activity or expression of the following enzymes (i) to (iv):
(i) at least one or more enzymes selected from the group consisting of Mtn, LuxS, and SahH (e.g., only SahH, or Mtn and LuxS),
(ii) at least one or more enzymes selected from the group consisting of ribose-phosphate diphosphokinase (Prs), adenine phosphoribosyltransferase (Apt), and adenosine kinase (ADK) (e.g., Prs and Apt or only ADK), and
(iii) at least one enzyme selected from the group consisting of MetF, methionine synthase (MetH or MetE), and MetK, and
(iv) at least one enzyme selected from the group consisting of aminomethyltransferase (GcvT), octanoyl-[GcvH]:protein N-octanoyltransferase (GcvH), and glycine dehydrogenase (GcvP) in addition to GlyA and SAM-dependent methyltransferase can be further used.

In the present invention, a cell engineered so as to enhance the activity or expression of the following enzymes (i) to (v):
(i) at least one or more enzymes selected from the group consisting of Mtn, LuxS, and SahH (e.g., only SahH, or Mtn and LuxS),
(ii) at least one or more enzymes selected from the group consisting of ribose-phosphate diphosphokinase (Prs), adenine phosphoribosyltransferase (Apt), and adenosine kinase (ADK) (e.g., Prs and Apt or only ADK), and
(iii) at least one enzyme selected from the group consisting of MetF, methionine synthase (MetH or MetE), and MetK,
(iv) at least one enzyme selected from the group consisting of GcvT, GcvH, and GcvP, and
(v) at least one enzyme selected from the group consisting of D-3-phosphoglycerate dehydrogenase (SerA), phosphoserine aminotransferase (SerC), and phosphoserine phosphatase (SerB)
in addition to GlyA and SAM-dependent methyltransferase can be further used.

Mtn, LuxS, SahH, Prs, Apt, ADK, MetF, methionine synthase (MetH or MetE), MetK, GcvT, GcvH, and GcvP are as described in the above section "2-1(4)."

GlyA is as described in the above section "2-2(4)."

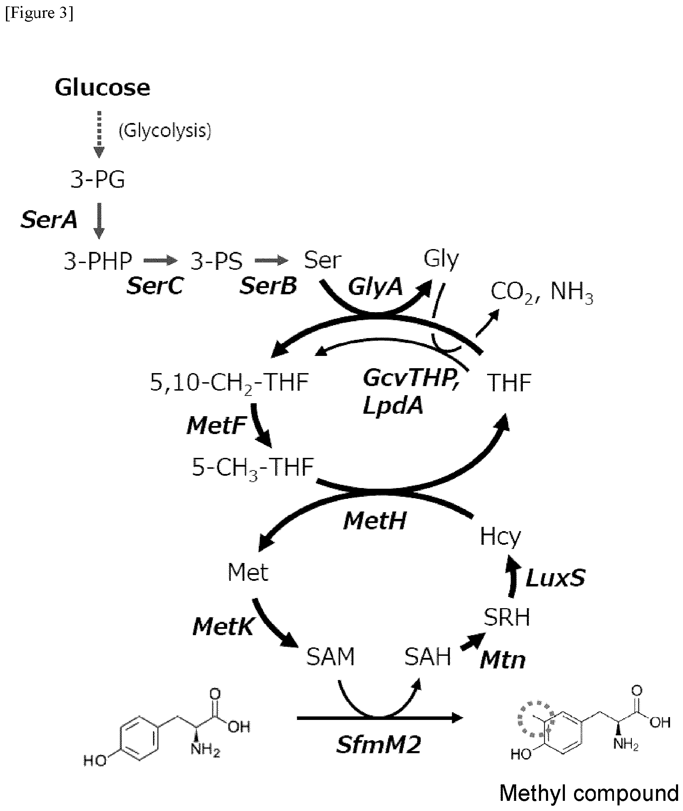

SerA is an enzyme that is utilized in the conversion of glyceraldehyde-3-phosphate (3-PG) to 3-phosphonooxypyruvate (3-PHP); SerC is an enzyme that is utilized in the conversion of 3-PHP to 3-phosphoserine (3-PS); and SerB is an enzyme that is utilized in the conversion of 3-PS to serine (Figure 3).

Examples of the cell engineered so as to enhance the activity or expression of GlyA and SAM-dependent methyltransferase include cells comprising a DNA encoding GlyA and a DNA encoding SAM-dependent methyltransferase. The SAM-dependent methyltransferase can be appropriately selected on the basis of a known database, for example, BRENDA (https://www.brenda-enzyme), as described above.

Examples of the cell engineered so as to enhance the activity or expression of each of Mtn, LuxS, SahH, Prs, Apt, ADK, MetF, MetH, MetE, MetK, GcvT, GcvH, GcvP, SerA, SerC, and SerB include cells comprising a DNA encoding each of Mtn, LuxS, SahH, Prs, Apt, ADK, MetF, MetH, MetE, MetK, GcvT, GcvH, GcvP, SerA, SerC, and SerB.

In the present invention, the "cell engineered" and the cell comprising a DNA encoding each enzyme can also be referred to as a "transformant."

In the present invention, the "microorganism engineered" and the microorganism comprising a DNA encoding each enzyme can also be referred to as a "transformant."

The DNA encoding each of SAM-dependent methyltransferase, Mtn, LuxS, SahH, Prs, Apt, ADK, MetF, MetH, MetE, MetK, GcvT, GcvH, GcvP, SerA, SerC, and SerB can be prepared by obtaining information on the nucleotide sequence thereof from a known database, for example, NCBI (https://www.ncbi.nlm.nih.gov/nucleotide/) and using a known genetic engineering approach.

In the present invention, a vector comprising the DNA encoding each enzyme can be used for transfecting a cell with the DNA. The type of the vector that can be used in the present invention is as described in the above section "2-1(4)."

In the present invention, the vector can comprise DNAs encoding one or more enzymes. For example, one vector may comprise DNAs encoding Mtn and LuxS, DNAs encoding MetF, MetH or MetE, MetK, Mtn, and LuxS, DNAs encoding MetF, MetH or MetE, and MetK, DNAs encoding Mtn and LuxS, DNAs encoding GcvT, GcvH, and GcvP, DNAs encoding Prs and Apt, DNAs encoding Prs, Apt, and ADK, or a DNA encoding ADK.

In the present invention, for example, a reaction system that converts serine originating from an organic raw material to glycine and also converts tetrahydrofolate to 5,10-methylenetetrahydrofolate can be introduced to a cell by introducing a DNA encoding GlyA to the cell.

In the present invention, for example, a glycine cleavage reaction system can be introduced to a cell by introducing DNAs encoding GcvT, GcvH, and GcvP to the cell. In the present invention, for example, a glycine cleavage reaction system may be introduced to a cell by introducing DNAs encoding GcvT, GcvH, and GcvP, as well as LpdA, to the cell.

The culture conditions of the cell used in the present invention are as described in the above section "2-1(4)."

### (4) Methyl compound

The "methyl compound" according to the present invention is as described in the above section "2-1(5)."

### (5) Method for producing a methyl compound

In the present invention, the method for producing a methyl compound comprises the step of performing methylation reaction using a cell engineered so as to enhance the activity or expression of serine hydroxymethyltransferase (GlyA) and S-adenosylmethionine-dependent methyltransferase. The term "cell" is as described in the above section "2-1(4)."

In the method for producing a methyl compound according to the present invention, the cell described in the above section "2-3(3)" can be used.

In the present invention, the method for producing a methyl compound comprises the step of allowing an organic raw material to act on the cell described in the above section "2.(3) Cell." As used herein, the phrase "allowing an organic raw material to act on the cell" means that the cell is cultured in the presence of an organic raw material.

The "organic raw material" as used herein is as described in the above section "2-1(4)."

The method for producing a methyl compound according to the present invention may comprise, for example, the steps of:
(a) preparing and culturing a cell comprising at least one DNA selected from (i) a DNA encoding GlyA, (ii) a DNA encoding SAM-dependent methyltransferase, (iii) DNAs encoding at least one or more enzymes selected from the group consisting of Mtn, LuxS, and SahH (e.g., only SahH, or Mtn and LuxS), (iv) DNAs encoding at least one or more enzymes selected from the group consisting of ribose-phosphate diphosphokinase (Prs), adenine phosphoribosyltransferase (Apt), and adenosine kinase (ADK), (v) DNAs encoding at least one or more enzymes selected from the group consisting of MetF, methionine synthase (MetH or MetE), and MetK, (vi) DNAs encoding at least one or more enzymes selected from the group consisting of GcvT, GcvH, and GcvP, and (vii) DNAs encoding at least one or more enzymes selected from the group consisting of SerA, SerC, and SerB; and
(b) adding an organic raw material to a culture solution of the cell obtained in step (a), followed by further culture.

The production method of the present invention can further comprise the step of recovering a produced methyl compound.

The production method of the present invention can further comprise the step of purifying a produced methyl compound.

Those skilled in the art can recover and purify the produced methyl compound by a known method suitable for the physical properties of the methyl compound, for example, treatment such as distillation, membrane dehydration, salting-out using an ion-exchange resin or the like, crystallization, or column chromatography.

In the present invention, the method for producing a methyl compound, in the case of using *E. coli* as a microorganism, can be performed, for example, as follows, though the method is not limited thereto.

First, the host *E. coli* is transfected with a DNA encoding each enzyme to prepare a transformant. The transformant is cultured overnight at approximately 37°C in a known culture medium (e.g., LB medium). Next, the transformant that has proliferated is suspended in a minimum medium (e.g., M9 medium), cultured at approximately 30°C for an appropriate time (e.g., 6 hours), then induced to express the gene using IPTG or the like, and further cultured for an appropriate time (e.g., 16 hours). A transformant with enhanced activity or expression of each enzyme can thereby be prepared.

Next, the transformant with enhanced activity or expression of each enzyme and an organic raw material (e.g., glucose)-containing solution (reaction buffer) are contacted with each other (e.g., the transformant is suspended in an organic raw material-containing solution). A methylation target substance is added thereto, and the transformant is further cultured at an appropriate temperature (e.g., approximately 30°C) for an appropriate time (e.g., approximately 6 to 24 hours) so that methylation reaction occurs to produce a methyl compound.

The organic raw material-containing solution can comprise, in addition to the organic raw material, for example, a buffer solution, and glucose as a source of ATP supply and may further comprise a divalent metal salt (e.g., magnesium sulfate).

### 3. Method for producing organic compound

In the present invention, an organic compound can be produced using a methyl compound produced by the method for producing a methyl compound according to the present invention as an intermediate. Specifically, the present invention provides a method for producing an organic compound, comprising the step of producing the organic compound via a methyl compound produced by the method for regenerating SAM or the method for producing a methyl compound according to the present invention as an intermediate.

As used herein, the organic compound means a compound produced via a methyl compound as an intermediate. Examples of the methyl compound for use as an intermediate include compounds produced by the method for regenerating SAM or the method for producing a methyl compound according to the present invention.

Those skilled in the art can produce an organic compound via the methyl compound as an intermediate on the basis of a known approach. Those skilled in the art can also appropriately set the production conditions thereof.

Examples of the organic compound that can be produced via the methyl compound as an intermediate include o-cresol, 2,6-xylenol, cumic acid, and terephthalic acid. Tyrosine is methylated by the method for regenerating SAM or the method for producing a methyl compound according to the present invention to produce 3-methyltyrosine or 3,5-dimethyltyrosine, and such a methyl compound can then be converted to o-cresol or 2,6-xylenol using lyase that recognizes each compound as a substrate. Perillic acid is methylated by the method for regenerating SAM or the method for producing a methyl compound according to the present invention to produce methyl perillate, which can then be converted to cumic acid and further to terephthalic acid through known chemical synthesis reaction (ChemistryOpen. 2018 Feb; 7 (2): 201-203). L-Histidine is methylated by the method for producing a methyl compound according to the present invention, and ergothioneine can be produced as an organic compound via hercynine as an intermediate.

Hereinafter, the present invention will be described in detail with reference to Examples. However, the present invention is not limited to these Examples. In the following Examples, the term "enhancement" or "enhanced" means that the activity or expression of an enzyme was enhanced.

### [Examples]

### [Example 1]

The expression of sfmM2 gene was enhanced in *E*. *coli* HMS 174(DE3) strain as follows.

### (A) Construction of SfmM2 enhancement plasmid

A DNA having a nucleotide sequence (sequence with recognition sequences of restriction enzymes Nde I and Bgl II added to the 5'- and 3'-terminal sides, respectively) comprising *Streptomyces lavendulae-*derived sfmM2 gene encoding tyrosine 3C-methyltransferase was synthesized (nucleotide sequence of synthetic DNA: SEQ ID NO: 1). The nucleotide sequence of the sfmM2 gene was designed as a codon-optimized sequence for efficient expression in *E. coli.* The synthesized DNA was inserted to restriction enzyme sites Nde I and Bgl II of an expression vector pET Duet-1 for *E. coli* (Novagen) and linked downstream of T7 promoter. The constructed plasmid was designated as pET_P_{T7}-sfmM2.

### (B) Preparation of SfmM2-enhanced strain

*E. coli* HMS174(DE3) strain (Novagen) was transformed with the plasmid pET_P_{T7}-sfmM2 constructed in Example 1(A), pCDF Duet-1 (Novagen), and pACYC Duet-1 (Novagen). The obtained strain was designated as HMS174(DE3)/pET_P_{T7}-sfmM2.

### [Example 2]

The expression of sfmM2 gene, gcvT gene, gcvH gene, and gcvP gene was enhanced in *E. coli* HMS174(DE3) strain as follows.

### (A) Construction of GcvTHP enhancement plasmid

A DNA having a nucleotide sequence (sequence with recognition sequences of restriction enzymes Nco I and BamHI added to the 5'- and 3'-terminal sides, respectively) comprising *Escherichia coli*-derived gcvT gene, gcvH gene, and gcvP gene (GcvT, GcvH, and GcvP are also collectively referred to as "GcvTHP") was synthesized (nucleotide sequence of synthetic DNA: SEQ ID NO: 2). The synthesized DNA was inserted to restriction enzyme sites Nco I and BamHI of an expression vector pACYC Duet-1 for *E*. *coli* (Novagen) and linked downstream of T7 promoter. The constructed plasmid was designated as pACYC_P_{T7}-gcvTHP.

### (B) Preparation of SfmM2, GcvTHP-enhanced strain

*E. coli* HMS174(DE3) strain (Novagen) was transformed with the plasmid pET_P_{T7}-sfmM2 constructed in Example 1(A), the plasmid pACYC_P_{T7}-gcvTHP constructed in Example 2(A), and pCDF Duet-1 (Novagen). The obtained strain was designated as HMS174(DE3)/pET_P_{T7}-sfmM2/pACYC_P_{T7}-gcvTHP.

### [Example 3]

The expression of sfmM2 gene and luxS gene was enhanced in *E. coli* HMS174(DE3) strain as follows.

### (A) Construction of Mtn, LuxS enhancement plasmid

A DNA having a nucleotide sequence (sequence with recognition sequences of restriction enzymes Nde I and Bgl II added to the 5'- and 3'-terminal sides, respectively) comprising *Escherichia coli*-derived mtn gene and luxS gene was synthesized (nucleotide sequence of synthetic DNA: SEQ ID NO: 3). The synthesized DNA was inserted to restriction enzyme sites Nde I and Bgl II of an expression vector pCDF Duet-1 for *E. coli* (Novagen) and linked downstream of T7 promoter. The constructed plasmid was designated as pCDF_P_{T7}-mtn-luxS.

### (B) Construction of LuxS enhancement plasmid

The plasmid pCDF_P_{T7}-mtn-luxS constructed in Example 2(A) was treated with a restriction enzyme Nde I, separated by agarose gel electrophoresis, then recovered from a gel, and purified to obtain a DNA fragment from which the mtn gene region was removed. Both ends of the obtained DNA fragment were self-ligated for linking. The constructed plasmid was designated as pCDF_P_{T7}-luxS.

### (C) Preparation of SfmM2, LuxS-enhanced strain

*E. coli* HMS174(DE3) strain (Novagen) was transformed with the plasmid pET_P_{T7}-sfmM2 constructed in Example 1(A), the plasmid pCDF_P_{T7}-luxS constructed in Example 3(B), and pACYC Duet-1 (Novagen). The obtained strain was designated as HMS174(DE3)/pET_P_{T7}-sfmM2/pCDF_P_{T7}-luxS.

### [Example 4]

The expression of sfmM2 gene, luxS gene, gcvT gene, gcvH gene, and gcvP gene was enhanced in *E. coli* HMS174(DE3) strain as follows.

*E. coli* HMS174(DE3) strain (Novagen) was transformed with the plasmid pET_P_{T7}-sfmM2 constructed in Example 1(A), the plasmid pCDF_P_{T7}-luxS constructed in Example 3(B), and the plasmid pACYC_P_{T7}-gcvTHP constructed in Example 2(A). The obtained strain was designated as HMS174(DE3)/pET_P_{T7}-sfmM2/pCDF_P_{T7}-luxS/pACYC_P_{T7}-gcvTHP.

### [Example 5]

The expression of sfmM2 gene, mtn gene, and luxS gene was enhanced in *E. coli* HMS174(DE3) strain as follows.

*E. coli* HMS174(DE3) strain (Novagen) was transformed with the plasmid pET_P_{T7}-sfmM2 constructed in Example 1(A), the plasmid pCDF_P_{T7}-mtn-luxS constructed in Example 3(A), and pACYC Duet-1 (Novagen). The obtained strain was designated as HMS174(DE3)/pET_P_{T7}-sfmM2/pCDF_P_{T7}-mtn-luxS.

### [Example 6]

The expression of sfmM2 gene, mtn gene, luxS gene, gcvT gene, gcvH gene, and gcvP gene was enhanced in *E. coli* HMS174(DE3) strain as follows.

*E. coli* HMS174(DE3) strain (Novagen) was transformed with the plasmid pET_P_{T7}-sfmM2 constructed in Example 1(A), the plasmid pCDF_P_{T7}-mtn-luxS constructed in Example 3(A), and the plasmid pACYC_P_{T7}-gcvTUP constructed in Example 2(A). The obtained strain was designated as HMS174(DE3)/pET_P_{T7}-sfmM2/pCDF_P_{T7}-mtn-luxS/pACYC_P_{T7}-gcvTHP.

### [Example 7]

The expression of sfmM2 gene and sahH gene was enhanced in *E. coli* HMS174(DE3) strain as follows.

### (A) Construction of SahH enhancement plasmid

A DNA having a nucleotide sequence (sequence with recognition sequences of restriction enzymes Nde I and Bgl II added to the 5'- and 3'-terminal sides, respectively) comprising *Pseudomonas aeruginosa*-derived sahH gene was synthesized (nucleotide sequence of synthetic DNA: SEQ ID NO: 4). The sahH gene was designed so as to have a codon-optimized sequence for efficient expression in *E. coli.* The synthesized DNA was inserted to restriction enzyme sites Nde I and Bgl II of an expression vector pCDF Duet-1 for *E. coli* (Novagen) and linked downstream of T7 promoter. The constructed plasmid was designated as pCDF_P_{T7}-sahH.

### (B) Preparation of SfmM2, SahH-enhanced strain

*E. coli* HMS174(DE3) strain (Novagen) was transformed with the plasmid pET_P_{T7}-sfmM2 constructed in Example 1(A), the plasmid pCDF_P_{T7}-sahH constructed in Example 7(A), and pACYC Duet-1 (Novagen). The obtained strain was designated as HMS174(DE3)/pET_P_{T7}-sfmM2/pCDF_P_{T7}-sahH.

### [Example 8]

The expression of sfmM2 gene, sahH gene, gcvT gene, gcvH gene, and gcvP gene was enhanced in *E. coli* HMS174(DE3) strain as follows.

*E. coli* HMS174(DE3) strain (Novagen) was transformed with the plasmid pET_P_{T7}-sfmM2 constructed in Example 1(A), the plasmid pCDF_P_{T7}-sahH constructed in Example 7(A), and the plasmid pACYC_P_{T7}-gcvTHP constructed in Example 2(A). The obtained strain was designated as HMS174(DE3)/pET_P_{T7}-sfmM2/pCDF_P_{T7}-sahH/pACYC_P_{T7}-gcvTHP.

### [Example 9]

The SfmM2-enhanced strain prepared in Example 1(B) was evaluated for 3-methyltyrosine production as follows.

HMS174(DE3)/pET_P₁₇-sfmM2 prepared in Example 1(B) was cultured overnight in LB medium (16 g/L Bacto-trypton, 10 g/L Yeast extract, 5 g/L NaCl) comprising 50 µg/mL carbenicillin, 50 µg/mL spectinomycin, and 34 µg/mL chloramphenicol. 5 mL of M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 0.5% glucose, 50 µg/mL carbenicillin, 50 µg/mL spectinomycin, and 34 µg/mL chloramphenicol was added to a 50 mL tube, and the culture solution of the LB medium was inoculated at a dilution ratio of 1/50 to the M9 medium, followed by shake culture at 200 rpm at 30°C for 6 hours. IPTG was added thereto at a final concentration of 0.5 mM, followed by further shake culture for 16 hours.

The obtained culture solution was centrifuged at 3000 × g for 5 minutes for harvest and suspended at OD₆₀₀ = 10 in a reaction buffer (100 mM Tris-HCl pH 8.0, 20 mM MgSO₄, 20 mM glucose) comprising 20 mM glycine, 100 µg/mL carbenicillin, 100 µg/mL spectinomycin, 68 µg/mL chloramphenicol, and 1 mM IPTG. 1 mL of the cell suspension was added to a 14 mL tube and mixed with 1 mL of a 10 mM L-tyrosine solution (dissolved in 0.1 N hydrochloric acid) serving as a substrate to start methylation reaction. The reaction solution was shaken at 200 rpm at 30°C for 24 hours. In order to make sure that methylation reaction could be accelerated by efficient regeneration of methyl group carbon of SAM, a test conducted under the same conditions as above except that glycine serving as a source of methyl group carbon supply was not added was also carried out, and the obtained results were compared with the results of the test conducted under the glycine addition conditions.

20 µL of the obtained reaction solution was mixed with 20 µL of pure water, 80 µL of 1 N hydrochloric acid, and 80 µL of acetonitrile, and a supernatant was collected after centrifugation at 3000 × g for 5 minutes and subjected to LC-MS analysis. The conditions of LC-MS analysis are shown in Table 1.

As a result of measurement, a 3-methyltyrosine concentration of the reaction solution was 0.340 mM under the glycine addition conditions, which was increased by 0.212 mM as compared with the glycine non-addition conditions.

**[Table 1]**

| | |
|---|---|
| Apparatus | Waters UPLC-MS (SQD2) |
| Column | Acquity UPLC BEH C18 1.7 µm 2.1 × 50 mm |
| Column temperature | 40°C |
| Mobile phase | (A) 0.1% aqueous formic acid solution |
| | (B) Acetonitrile containing 0.1% formic acid |
| Detection | PDA (245-400 nm), MS (ESI+/-) |
| Flow rate | 0.2 mL/min |
| Elution (B%) | Held at 0% for 1 min, elevated to 90% over 4.5 min, held at 90% for 1 min. |
| | Lowered to 0% over 0.5 min, and then held at 0% for 3 min. |

### [Example 10]

The SfmM2-enhanced strain prepared in Example 1(B) was evaluated for 3-methyltyrosine production under conditions involving glycine addition at the time of culture as follows.

In this Example, the 3-methyltyrosine production was evaluated in the same manner as in Example 9 except that after culture in LB medium, M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 10 mM glycine, 0.5% glucose, 50 µg/mL carbenicillin, 50 µg/mL spectinomycin, and 34 µg/mL chloramphenicol was used.

As a result, a 3-methyltyrosine concentration of the reaction solution was 0.442 mM under the glycine addition conditions, which was increased by 0.267 mM as compared with the glycine non-addition conditions.

### [Example 11]

The SfmM2, GcvTHP-enhanced strain prepared in Example 2(B) was evaluated for 3-methyltyrosine production as follows.

In this Example, the 3-methyltyrosine production was evaluated in the same manner as in Example 9 except that HMS174(DE3)/pET_P_{T7}-sfmM2/pACYC_P_{T7}-gcvTHP prepared in Example 2(B) was used.

As a result, a 3-methyltyrosine concentration of the reaction solution was 0.196 mM under the glycine addition conditions, which was increased by 0.099 mM as compared with the glycine non-addition conditions.

### [Example 12]

The SfmM2, GcvTHP-enhanced strain prepared in Example 2(B) was evaluated for 3-methyltyrosine production under conditions involving glycine addition at the time of culture as follows.

In this Example, the 3-methyltyrosine production was evaluated in the same manner as in Example 9 except that HMS174(DE3)/pET_P_{T7}-sfmM2/pACYC_P_{T7}-gcvTHP prepared in Example 2(B) was used; and after culture in LB medium, M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 10 mM glycine, 0.5% glucose, 50 µg/mL carbenicillin, 50 µg/mL spectinomycin, and 34 µg/mL chloramphenicol was used.

As a result, a 3-methyltyrosine concentration of the reaction solution was 0.470 mM under the glycine addition conditions, which was increased by 0.310 mM as compared with the glycine non-addition conditions.

### [Example 13]

The SfmM2, LuxS -enhanced strain prepared in Example 3(C) was evaluated for 3-methyltyrosine production under conditions involving glycine addition at the time of culture as follows.

In this Example, the 3-methyltyrosine production was evaluated in the same manner as in Example 9 except that HMS174(DE3)/pET_P_{T7}-sfmM2/pCDF_P_{T7}-LuxS prepared in Example 3(C) was used; and after culture in LB medium, M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 10 mM glycine, 0.5% glucose, 50 µg/mL carbenicillin, 50 µg/mL spectinomycin, and 34 µg/mL chloramphenicol was used.

As a result, a 3-methyltyrosine concentration of the reaction solution was 0.536 mM under the glycine addition conditions, which was increased by 0.215 mM as compared with the glycine non-addition conditions.

### [Example 14]

The SfmM2, LuxS, GcvTHP -enhanced strain prepared in Example 4 was evaluated for 3-methyltyrosine production under conditions involving glycine addition at the time of culture as follows.

In this Example, the 3-methyltyrosine production was evaluated in the same manner as in Example 9 except that HMS174(DE3)/pET_P_{T7}-sfmM2/pCDF_P_{T7}-LuxS/pACYC_P_{T7}-gcvTHP prepared in Example 4 was used; and after culture in LB medium, M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 10 mM glycine, 0.5% glucose, 50 µg/mL carbenicillin, 50 µg/mL spectinomycin, and 34 µg/mL chloramphenicol was used.

As a result, a 3-methyltyrosine concentration of the reaction solution was 0.576 mM under the glycine addition conditions, which was increased by 0.142 mM as compared with the glycine non-addition conditions.

### [Example 15]

The SfmM2, Mtn, LuxS -enhanced strain prepared in Example 5 was evaluated for 3-methyltyrosine production as follows.

In this Example, the 3-methyltyrosine production was evaluated in the same manner as in Example 9 except that HMS174(DE3)/pET_P_{T7}-sfmM2/pCDF_P_{T7}-mtn-luxS prepared in Example 5 was used.

As a result, a 3-methyltyrosine concentration of the reaction solution was 0.558 mM under the glycine addition conditions, which was increased by 0.372 mM as compared with the glycine non-addition conditions.

### [Example 16]

The SfmM2, Mtn, LuxS -enhanced strain prepared in Example 5 was evaluated for 3-methyltyrosine production under conditions involving glycine addition at the time of culture as follows.

In this Example, the 3-methyltyrosine production was evaluated in the same manner as in Example 9 except that HMS174(DE3)/pET_P_{T7}-sfmM2/pCDF_P_{T7}-mtn-LuxS prepared in Example 5 was used; and after culture in LB medium, M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 10 mM glycine, 0.5% glucose, 50 µg/mL carbenicillin, 50 µg/mL spectinomycin, and 34 µg/mL chloramphenicol was used.

As a result, a 3-methyltyrosine concentration of the reaction solution was 0.787 mM under the glycine addition conditions, which was increased by 0.331 mM as compared with the glycine non-addition conditions.

### [Example 17]

The SfmM2, Mtn, LuxS, GcvTHP-enhanced strain prepared in Example 6 was evaluated for 3-methyltyrosine production as follows.

In this Example, the 3-methyltyrosine production was evaluated in the same manner as in Example 9 except that HMS174(DE3)/pET_P_{T7}-sfmM2/pCDF_P_{T7}-mtn-luxS/pACYC_P_{T7}-gcvTHP prepared in Example 6 was used.

As a result, a 3-methyltyrosine concentration of the reaction solution was 0.258 mM under the glycine addition conditions, which was increased by 0.150 mM as compared with the glycine non-addition conditions.

### [Example 18]

The SfmM2, Mtn, LuxS, GcvTHP -enhanced strain prepared in Example 6 was evaluated for 3-methyltyrosine production under conditions involving glycine addition at the time of culture as follows.

In this Example, the 3-methyltyrosine production was evaluated in the same manner as in Example 9 except that HMS174(DE3)/pET_P_{T7}-sfmM2/pCDF_P_{T7}-mtn-LuxS/pACYC_P_{T7}-gcvTUP prepared in Example 6 was used; and after culture in LB medium, M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 10 mM glycine, 0.5% glucose, 50 µg/mL carbenicillin, 50 µg/mL spectinomycin, and 34 µg/mL chloramphenicol was used.

As a result, a 3-methyltyrosine concentration of the reaction solution was 0.829 mM under the glycine addition conditions, which was increased by 0.567 mM as compared with the glycine non-addition conditions.

### [Example 19]

The SfmM2, sahH -enhanced strain prepared in Example 7(B) was evaluated for 3-methyltyrosine production under conditions involving glycine addition at the time of culture as follows.

In this Example, the 3-methyltyrosine production was evaluated in the same manner as in Example 9 except that HMS174(DE3)/pET_P_{T7}-sfmM2/pCDF_P_{T7}-sahH prepared in Example 7(B) was used; and after culture in LB medium, M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 10 mM glycine, 0.5% glucose, 50 µg/mL carbenicillin, 50 µg/mL spectinomycin, and 34 µg/mL chloramphenicol was used.

As a result, a 3-methyltyrosine concentration of the reaction solution was 0.284 mM under the glycine addition conditions, which was increased by 0.196 mM as compared with the glycine non-addition conditions.

### [Example 20]

The SfmM2, SahH, GcvTHP -enhanced strain prepared in Example 8 was evaluated for 3-methyltyrosine production under conditions involving glycine addition at the time of culture as follows.

In this Example, the 3-methyltyrosine production was evaluated in the same manner as in Example 9 except that HMS174(DE3)/pET_P_{T7}-sfmM2/pCDF_P_{T7}-sahH/pACYC_P_{T7}-gcvTHP prepared in Example 8 was used; and after culture in LB medium, M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 10 mM glycine, 0.5% glucose, 50 µg/mL carbenicillin, 50 µg/mL spectinomycin, and 34 µg/mL chloramphenicol was used.

As a result, a 3-methyltyrosine concentration of the reaction solution was 0.313 mM under the glycine addition conditions, which was increased by 0.219 mM as compared with the glycine non-addition conditions.

The results of Examples 9 to 12 and 15 to 18 were summarized in Table 2. In these Examples, the concentrations of accumulated 3-methyltyrosine under conditions involving glycine addition at the time of methylation reaction were drastically increased to 2.7 times, 2.5 times, 2.0 times, 2.9 times, 3.0 times, 1.7 times, 2.4 times, and 3.2 times, respectively, as compared with those under glycine non-addition conditions. These results demonstrated that performing methylation reaction in the presence of glycine achieves efficient regeneration of SAM and can accelerate the methylation reaction and increase the amount of an arbitrary methyl compound produced.

In Examples 10, 12, 16, and 18, the concentrations of accumulated 3-methyltyrosine under conditions involving glycine addition at the time of methylation reaction were 1.3 times, 2.4 times, 1.4 times, and 3.2 times, respectively, those in Examples 9, 11, 15, and 17. These results demonstrated that the preparation of microbial cells by the addition of glycine at the time of culture (before the start of methylation reaction) improves SAM regeneration efficiency and can further accelerate the methylation reaction and increase the amount of an arbitrary methyl compound produced.

Specifically, the present invention was found able to markedly accelerate methylation reaction by culturing cells in the presence of an organic raw material comprising glycine (adding glycine before addition of a methylation target substance which is before the start of methylation reaction).

**[Table 2]**

| | Microbial strain | Added component at time of culture | Source of methyl group supply | Concentration of accumulated 3- methyltyrosine [mM] | Amount of increase in concentration of accumulated 3-methyltyrosine as compared with glycine non-addition conditions [mM] | Relative value of concentration of accumulated 3-methyltyrosine as compared with glycine non-addition conditions |
|---|---|---|---|---|---|---|
| Example 9 | HMS174 (DE3)/pET_P_{T7}-sfmM2 | - | Glycine | 0.340 | 0.212 | 2.7 |
| Example 10 | | Glycine | Glycine | 0.442 | 0.267 | 2.5 |
| Example 11 | HMS174 (DE3)/pET _P_{T7}-sfmM2/pACYC_P_{T 7}-gcvTHP | - | Glycine | 0.196 | 0.099 | 2.0 |
| Example 12 | | Glycine | Glycine | 0.470 | 0.310 | 2.9 |
| Example 15 | HMS174 (DE3)/pET _P_{T7}-sfmM2/pCDF_P_{T7}-mtn-luxS | - | Glycine | 0.558 | 0.372 | 3.0 |
| Example 16 | | Glycine | Glycine | 0.787 | 0.331 | 1.7 |
| Example 17 | HMS174(DE3)/pET P_{T7}-sfmM2/pCDF_P_{T7}-mtn-luxS/pACYC_P_{T7}-gcvTHP | - | Glycine | 0.258 | 0.150 | 2.4 |
| Example 18 | | Glycine | Glycine | 0.829 | 0.567 | 3.2 |

The results of Examples 10, 12 to 14, 16, and 18 to 20 were summarized in Table 3. In Examples 13, 14, 19, and 20, the concentrations of accumulated 3-methyltyrosine under conditions involving glycine addition at the time of methylation reaction were drastically increased to 1.7 times, 1.3 times, 3.2 times, and 3.3 times, respectively, as compared with those under glycine non-addition conditions. These results demonstrated that in these microbial strains as well, performing methylation reaction in the presence of glycine achieves efficient regeneration of SAM and can accelerate the methylation reaction.

In Examples 12, 14, 18, and 20, the concentrations of accumulated 3-methyltyrosine under conditions involving glycine addition at the time of methylation reaction were increased by 6.3%, 7.4%, 5.3%, and 10%, respectively, as compared with Examples 10, 13, 16, and 19. These results demonstrated that the enhancement of GcvTHP improves SAM regeneration efficiency and can further accelerate the methylation reaction and increase the amount of an arbitrary methyl compound produced.

In Examples 13 and 16, the concentration of accumulated 3-methyltyrosine under conditions involving glycine addition at the time of methylation reaction was increased by 21% and 78%, respectively, as compared with Example 10. In Examples 14, and 18, the concentration of accumulated 3-methyltyrosine under conditions involving glycine addition at the time of methylation reaction was increased by 23% and 76%, respectively, as compared with Example 12. These results demonstrated that the enhancement of LuxS or the enhancement of Mtn and LuxS improves SAM regeneration efficiency and can further accelerate the methylation reaction and increase the amount of an arbitrary methyl compound produced.

Specifically, the method of the present invention was found able to efficiently regenerate SAM, to accelerate methylation reaction, and to increase the amount of an arbitrary methyl compound produced, by using glycine.

**[Table 3]**

| | Microbial strain | Added component at time of culture | Source of methyl group supply | Concentration of accumulated 3-methyltyrosine [mM] | Amount of increase in concentration of accumulated 3-methyltyrosine as compared with glycine non-addition conditions [mM] | Relative value of concentration of accumulated 3-methyltyrosine as compared with glycine non-addition conditions |
|---|---|---|---|---|---|---|
| Example 10 | HMS174 (DE3)/pET_P_{T7} - sfmM2 | Glycine | Glycine | 0.442 | 0.267 | 2.5 |
| Example 12 | HMS174 (DE3)/pET_P_{T7} - sfmM2/pACYC_P_{T7} -gcvTHP | Glycine | Glycine | 0.470 | 0.310 | 2.9 |
| Example 13 | HMS174 (DE3)/pET_P_{T7} - sfmM2/pCDF_P_{T7}-luxS | Glycine | Glycine | 0.536 | 0.215 | 1.7 |
| Example 14 | HMS174 (DE3)/pET_P_{T7} - sfmM2/pCDF_P_{T7}-luxS/pACYC_P_{T7}-gcvTHP | Glycine | Glycine | 0.576 | 0.142 | 1.3 |
| Example 16 | HMS174 (DE3)/pET_P_{T7} - sfmM2/pCDF_P_{T7}-mtn-luxS | Glycine | Glycine | 0.787 | 0.331 | 1.7 |
| Example 18 | HMS174 (DE3)/pET_P_{T7} - sfmM2/pCDF_P_{T7}-mtn-luxS/pACYC_P_{T7}-gcvTHP | Glycine | Glycine | 0.829 | 0.567 | 3.2 |
| Example 19 | HMS174 (DE3)/pET_P_{T7} - sfmM2/pCDF_P_{T7}-sahH | Glycine | Glycine | 0.284 | 0.196 | 3.2 |
| Example 20 | HMS174 (DE3)/pET_P_{T7} - sfmM2/pCDF_P_{T7}-sahH/pACYC_P_{T7}-gcvTHP | Glycine | Glycine | 0.313 | 0.219 | 3.3 |

### [Example 21]

The expression of TCMT gene was enhanced in *E. coli* HMS174(DE3) strain as follows.

### (A) Construction of TCMT enhancement plasmid

A DNA having a nucleotide sequence (sequence with recognition sequences of restriction enzymes Nde I and Bgl II added to the 5'- and 3'-terminal sides, respectively) comprising *Lonsdalea populi-derived* TCMT gene encoding tyrosine 3C-methyltransferase was synthesized (nucleotide sequence of synthetic DNA: SEQ ID NO: 5). The nucleotide sequence of the TCMT gene was designed as a codon-optimized sequence for efficient expression in *E. coli.* The synthesized DNA was inserted to restriction enzyme sites Nde I and Bgl II of an expression vector pET Duet-1 for *E. coli* (Novagen) and linked downstream of T7 promoter. The constructed plasmid was designated as pET_P_{T7}-TCMT.

### (B) Preparation of TCMT-enhanced strain

*E. coli* HMS174(DE3) strain (Novagen) was transformed with the plasmid pET_P_{T7}-TCMT constructed in Example 21(A), pCDF Duet-1 (Novagen), and pACYC Duet-1 (Novagen). The obtained strain was designated as HMS174(DE3)/pET_P_{T7}-TCMT.

### [Example 22]

The expression of TCMT gene, mtn gene, and luxS gene was enhanced in *E. coli* HMS174(DE3) strain as follows.

*E. coli* HMS174(DE3) strain (Novagen) was transformed with the plasmid pET_P_{T7}-TCMT constructed in Example 21(A), the plasmid pCDF_P_{T7}-mtn-luxS constructed in Example 3(A), and pACYC Duet-1 (Novagen). The obtained strain was designated as HMS174(DE3)/pET_P_{T7}-TCMT/pCDF_P_{T7}-mtn-luxS.

### [Example 23]

The expression of TCMT gene, mtn gene, luxS gene, prs gene, and apt gene was enhanced in *E. coli* HMS174(DE3) strain as follows.

### (A) Construction of Mtn, LuxS, Prs, Apt enhancement plasmid

A DNA having a nucleotide sequence (sequence with recognition sequences of restriction enzymes Bgl II and Fse I added to the 5'- and 3'-terminal sides, respectively) comprising *Escherichia* coli-derived prs gene and apt gene was synthesized (nucleotide sequence of synthetic DNA: SEQ ID NO: 6). The synthesized DNA was inserted to restriction enzyme sites Bgl II and Fse I of the plasmid pCDF_P_{T7}-mtn-luxS constructed in Example 3(A), and linked downstream of the luxS gene. The constructed plasmid was designated as pCDF_P_{T7}-mtn-luxS-prs-apt.

### (B) Preparation of TCMT, Mtn, LuxS, Prs, Apt-enhanced strain

*E. coli* HMS174(DE3) strain (Novagen) was transformed with the plasmid pET_P_{T7}-TCMT constructed in Example 21(A), the plasmid pCDF_P_{T7}-mtn-luxS-prs-apt constructed in Example 23(A), and pACYC Duet-1 (Novagen). The obtained strain was designated as HMS174(DE3)/pET_P_{T7}-TCMT/pCDF_P_{T7}-mtn-luxS-prs-apt.

### [Example 24]

The expression of TCMT gene, mtn gene, luxS gene, prs gene, apt gene, gcvT gene, gcvH gene, and gcvP gene was enhanced in *E. coli* HMS174(DE3) strain as follows.

*E. coli* HMS174(DE3) strain (Novagen) was transformed with the plasmid pET_P_{T7}-TCMT constructed in Example 21(A), the plasmid pCDF_P_{T7}-mtn-luxS-prs-apt constructed in Example 23(A), and the plasmid pACYC_P_{T7}-gcvTUP constructed in Example 2(A). The obtained strain was designated as HMS174(DE3)/pET_P_{T7}-TCMT/pCDF_P_{T7}-mtn-luxS-prs-apt/pACYC_P_{T7}-gcvTHP.

### [Example 25]

The expression of TCMT gene, sahH gene, gcvT gene, gcvH gene, and gcvP gene was enhanced in *E. coli* HMS174(DE3) strain as follows.

*E. coli* HMS174(DE3) strain (Novagen) was transformed with the plasmid pET_P_{T7}-TCMT constructed in Example 21(A), the plasmid pCDF_P_{T7}-sahH constructed in Example 7(A), and the plasmid pACYC_P_{T7}-gcvTHP constructed in Example 2(A). The obtained strain was designated as HMS174(DE3)/pET_P_{T7}-TCMT/pCDF_P_{T7}-sahH/pACYC_P_{T7}-gcvTHP.

### [Example 26]

The expression of TCMT gene, sahH gene, ADO1 gene, gcvT gene, gcvH gene, and gcvP gene was enhanced in *E. coli* HMS174(DE3) strain as follows. The ADO1 gene is a gene encoding adenosine kinase (ADK).

### (A) Construction of SahH-ADO1 enhancement plasmid

A DNA having a nucleotide sequence (sequence with recognition sequences of restriction enzymes Bgl II and Mfe I added to the 5'- and 3'-terminal sides, respectively) comprising *Saccharomyces cerevisiae-derived* ADO1 gene was synthesized (nucleotide sequence of synthetic DNA: SEQ ID NO: 7). The nucleotide sequence of the ADO1 gene was designed as a codon-optimized sequence for efficient expression in *E. coli.* The synthesized DNA was inserted to restriction enzyme sites Bgl II and Mfe I of the plasmid pCDF_P_{T7}-sahH constructed in Example 7(A), and linked downstream of the sahH gene. The constructed plasmid was designated as pCDF_P_{T7}-sahH-ADO1.

### (B) Preparation of TCMT, SahH, ADO1, GcvTHP-enhanced strain

*E. coli* HMS174(DE3) strain (Novagen) was transformed with the plasmid pET_P_{T7}-TCMT constructed in Example 21(A), the plasmid pCDF_P_{T7}-sahH-ADO1 constructed in Example 26(A), and the plasmid pACYC_P_{T7}-gcvTUP constructed in Example 2(A). The obtained strain was designated as HMS174(DE3)/pET_P_{T7}-TCMT/pCDF_P_{T7}-sahH-ADO1/pACYC_P_{T7}-gcvTHP.

### [Example 27]

The TCMT-enhanced strain prepared in Example 21(B) was evaluated for 3-methyltyrosine production as follows.

In this Example, the 3-methyltyrosine production was evaluated in the same manner as in Example 9 except that HMS174(DE3)/pET_P_{T7}-TCMT prepared in Example 21(B) was used; after culture in LB medium, M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 10 mM glycine, 0.5% glucose, 50 µg/mL carbenicillin, 50 µg/mL spectinomycin, and 34 µg/mL chloramphenicol was used; and the suspension in a reaction buffer was performed at OD₆₀₀ = 5.

As a result, a 3-methyltyrosine concentration of the reaction solution was 0.175 mM under the glycine addition conditions, which was increased by 0.154 mM as compared with the glycine non-addition conditions.

### [Example 28]

The TCMT, Mtn, LuxS-enhanced strain prepared in Example 22 was evaluated for 3-methyltyrosine production as follows.

In this Example, the 3-methyltyrosine production was evaluated in the same manner as in Example 27 except that HMS174(DE3)/pET_P_{T7}-TCMT/pCDF_P_{T7}-mtn-luxS prepared in Example 22 was used.

As a result, a 3-methyltyrosine concentration of the reaction solution was 0.481 mM under the glycine addition conditions, which was increased by 0.308 mM as compared with the glycine non-addition conditions.

### [Example 29]

The TCMT, Mtn, LuxS, Prs, Apt -enhanced strain prepared in Example 23(B) was evaluated for 3-methyltyrosine production as follows.

In this Example, the 3-methyltyrosine production was evaluated in the same manner as in Example 27 except that HMS174(DE3)/pET_P_{T7}-TCMT/pCDF_P_{T7}-mtn-luxS-prs-apt prepared in Example 23(B) was used.

As a result, a 3-methyltyrosine concentration of the reaction solution was 0.568 mM under the glycine addition conditions, which was increased by 0.352 mM as compared with the glycine non-addition conditions.

### [Example 30]

The TCMT, Mtn, LuxS, prs, Apt, GcvTHP -enhanced strain prepared in Example 24 was evaluated for 3-methyltyrosine production as follows.

In this Example, the 3-methyltyrosine production was evaluated in the same manner as in Example 27 except that HMS174(DE3)/pET_P_{T7}-TCMT/pCDF_P_{T7}-mtn-luxS-prs-apt/pACYC_P_{T7}-gcvTUP prepared in Example 24 was used.

As a result, a 3-methyltyrosine concentration of the reaction solution was 0.571 mM under the glycine addition conditions, which was increased by 0.227 mM as compared with the glycine non-addition conditions.

### [Example 31]

The TCMT, SahH, GcvTHP -enhanced strain prepared in Example 25 was evaluated for 3-methyltyrosine production as follows.

In this Example, the 3-methyltyrosine production was evaluated in the same manner as in Example 27 except that HMS174(DE3)/pET_P_{T7}-TCMT/pCDF_P_{T7}-sahH/pACYC_P_{T7}-gcvTHP prepared in Example 25 was used.

As a result, a 3-methyltyrosine concentration of the reaction solution was 0.189 mM under the glycine addition conditions, which was increased by 0.167 mM as compared with the glycine non-addition conditions.

### [Example 32]

The TCMT, SahH, ADO1, GcvTHP -enhanced strain prepared in Example 26(B) was evaluated for 3-methyltyrosine production as follows.

In this Example, the 3-methyltyrosine production was evaluated in the same manner as in Example 27 except that HMS174(DE3)/pET_P_{T7}-TCMT/pCDF_P_{T7}-sahH-ADO1/pACYC_P_{T7}-gcvTUP prepared in Example 26(B) was used.

As a result, a 3-methyltyrosine concentration of the reaction solution was 0.288 mM under the glycine addition conditions, which was increased by 0.226 mM as compared with the glycine non-addition conditions.

The results of Examples 27 to 32 were summarized in Table 4. In Examples 27, 28, 29, 30, 31, and 32, the concentrations of accumulated 3-methyltyrosine under conditions involving glycine addition at the time of methylation reaction were drastically increased to 8.3 times, 2.8 times, 2.6 times, 1.7 times, 8.6 times, and 4.6 times, respectively, as compared with those under glycine non-addition conditions. These results demonstrated that in these microbial strains as well, performing methylation reaction in the presence of glycine achieves efficient regeneration of SAM and can accelerate the methylation reaction.

In Examples 28, 29, and 30, the concentrations of accumulated 3-methyltyrosine under conditions involving glycine addition at the time of methylation reaction were increased to 2.7 times, 3.2 times, and 3.3 times, respectively, as compared with those in Example 27. These results demonstrated that the enhancement of Mtn, LuxS, Prs, Apt, and GcvTHP improves SAM regeneration efficiency and can further accelerate the methylation reaction and increase the amount of an arbitrary methyl compound produced.

In Examples 31 and 32, the concentration of accumulated 3-methyltyrosine under conditions involving glycine addition at the time of methylation reaction was increased by 8% and 65%, respectively, as compared with Example 27. These results demonstrated that the enhancement of SahH, ADO1, and GcvTHP improves SAM regeneration efficiency and can further accelerate the methylation reaction and increase the amount of an arbitrary methyl compound produced.

Specifically, the method of the present invention was found able to efficiently regenerate SAM, to accelerate methylation reaction, and to increase the amount of an arbitrary methyl compound produced, by using glycine.

**[Table 4]**

| | Microbial strain | Added compone nt at time of culture | Source of methyl group supply | Concentratio n of accumulated 3-methyltyrosi ne [mM] | Amount of increase in concentration of accumulated 3-methyltyrosi ne as compared with glycine non-addition conditions [mM] | Relative value of concentration of accumulated 3-methyltyrosi ne as compared with glycine non-addition conditions |
|---|---|---|---|---|---|---|
| Exampl e 27 | HMS174 (DE3)/pET_P_{T7}-TCMT | Glycine | Glycin e | 0.175 | 0.154 | 8.3 |
| Exampl e 28 | HMS174 (DE3)/pET_P_{T7}-TCMT/pCDF_P_{T7} -mtn-luxS | Glycine | Glycin e | 0.481 | 0.308 | 2.8 |
| Exampl e 29 | HMS174 (DE3)/pET_P_{T7}-TCMT/pCDF_P_{T7} -mtn-luxS-prs-apt | Glycine | Glycin e | 0.568 | 0.352 | 2.6 |
| Exampl e 30 | HMS174 (DE3)/pET_P_{T7}-TCMT/pCDF_P_{T7} -mtn-luxS-prs-apt/pACYC_P_{T7}-gcvTHP | Glycine | Glycin e | 0.571 | 0.227 | 1.7 |
| Exampl e 31 | HMS174 (DE3)/pET_P_{T7}-TCMT/pCDF_P_{T7} sahH/pACYC_P_{T7} -gcvTHP | Glycine | Glycin e | 0.189 | 0.167 | 8.6 |
| Exampl e 32 | HMS174 (DE3)/pET_P_{T7}-TCMT/pCDF_P_{T7} -sahH-AD01/pACYC_P_{T 7}-gcvTHP | Glycine | Glycin e | 0.288 | 0.226 | 4.6 |

### [Example 33]

The expression of EgtB gene, EgtD gene, and EgtE gene was enhanced in *E. coli* HMS174(DE3) strain as follows.

### (A) Construction of EgtBDE enhancement plasmid

A DNA having a nucleotide sequence (sequence with recognition sequences of restriction enzymes Nco I and EcoR I added to the 5'- and 3'-terminal sides, respectively) comprising *Methylobacterium pseudosasicola*-derived EgtB gene encoding hercynyl cysteine S-oxide synthase and *Mycolicibacterium smegmatis-derived* EgtD gene and EgtE gene encoding L-histidine Nα-methyltransferase and hercynyl cysteine S-oxide lyase, respectively, was synthesized (nucleotide sequence of synthetic DNA: SEQ ID NO: 8). The nucleotide sequences of the EgtB gene, the EgtD gene, and the EgtE gene were each designed as a codon-optimized sequence for efficient expression in *E. coli.* The synthesized DNA was inserted to restriction enzyme sites Nco I and EcoR I of an expression vector pET Duet-1 for *E. coli* (Novagen) and linked downstream of T7 promoter. The constructed plasmid was designated as pET_P_{T7}-EgtBDE.

### (B) Preparation of EgtBDE-enhanced strain

*E. coli* HMS174(DE3) strain (Novagen) was transformed with the plasmid pET_P_{T7}-EgtBDE constructed in Example 33(A), pCDF Duet-1 (Novagen), and pACYC Duet-1 (Novagen). The obtained strain was designated as HMS174(DE3)/pETP_{T7}-EgtBDE.

### [Example 34]

The expression of EgtB gene, EgtD gene, EgtE gene, mtn gene, and luxS gene was enhanced in *E. coli* HMS174(DE3) strain as follows.

*E. coli* HMS174(DE3) strain (Novagen) was transformed with the plasmid pET_P_{T7}-EgtBDE constructed in Example 33(A), the plasmid pCDF_P_{T7}-mtn-luxS constructed in Example 3(A), and pACYC Duet-1 (Novagen). The obtained strain was designated as HMS174(DE3)/pET_P_{T7}-EgtBDE/pCDF_P_{T7}-mtn-luxS.

### [Example 35]

The expression of EgtB gene, EgtD gene, EgtE gene, mtn gene, luxS gene, prs gene, and apt gene was enhanced in *E. coli* HMS174(DE3) strain as follows.

*E. coli* HMS174(DE3) strain (Novagen) was transformed with the plasmid pET_P_{T7}-EgtBDE constructed in Example 33(A), the plasmid pCDF_P_{T7}-mtn-luxS-prs-apt constructed in Example 23(A), and pACYC Duet-1 (Novagen). The obtained strain was designated as HMS174(DE3)/pET_P_{T7}-EgtBDE/pCDF_P_{T7}-mtn-luxS-prs-apt.

### [Example 36]

The expression of EgtB gene, EgtD gene, EgtE gene, mtn gene, luxS gene, prs gene, apt gene, gcvT gene, gcvH gene, and gcvP gene was enhanced in *E. coli* HMS174(DE3) strain as follows.

*E. coli* HMS174(DE3) strain (Novagen) was transformed with the plasmid pET_P_{T7}-EgtBDE constructed in Example 33(A), the plasmid pCDF_P_{T7}-mtn-luxS-prs-apt constructed in Example 23(A), and the plasmid pACYC_P_{T7}-gcvTUP constructed in Example 2(A). The obtained strain was designated as HMS174(DE3)/pET_P_{T7}-EgtBDE/pCDF_P_{T7}-mtn-luxS-prs-apt/pACYC_P_{T7}-gcvTHP.

### [Example 37]

The expression of EgtB gene, EgtD gene, EgtE gene, sahH gene, ADO1 gene, gcvT gene, gcvH gene, and gcvP gene was enhanced in *E. coli* HMS174(DE3) strain as follows.

*E. coli* HMS174(DE3) strain (Novagen) was transformed with the plasmid pET_P_{T7}-EgtBDE constructed in Example 33(A), the plasmid pCDF_P_{T7}-sahH-ADO1 constructed in Example 26(A), and the plasmid pACYC_P_{T7}-gcvTHP constructed in Example 2(A). The obtained strain was designated as HMS174(DE3)/pET_P_{T7}-EgtBDE/pCDF_P_{T7}-sahH-ADO1/pACYC_P_{T7}-gcvTHP.

### [Example 38]

The EgtBDE-enhanced strain prepared in Example 33(B) was evaluated for hercynine and ergothioneine production as follows.

HMS174(DE3)/pET_P_{T7}-EgtBDE prepared in Example 33(B) was cultured overnight in LB medium (16 g/L Bacto-trypton, 10 g/L Yeast extract, 5 g/L NaCl) comprising 50 µg/mL carbenicillin, 50 µg/mL spectinomycin, and 34 µg/mL chloramphenicol. 5 mL of M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 0.5% glucose, 50 µg/mL carbenicillin, 50 µg/mL spectinomycin, and 34 µg/mL chloramphenicol was added to a 50 mL tube, and the culture solution of the LB medium was inoculated at a dilution ratio of 1/50 to the M9 medium, followed by shake culture at 200 rpm at 30°C for 6 hours. IPTG was added thereto at a final concentration of 0.5 mM, followed by further shake culture for 16 hours.

The obtained culture solution was centrifuged at 3000 × g for 5 minutes for harvest and suspended at OD₆₀₀ = 4 in a reaction buffer (100 mM Tris-HCl pH 8.0, 20 mM MgSO₄, 20 mM glucose) comprising 20 mM glycine, 100 µg/mL carbenicillin, 100 µg/mL spectinomycin, 68 µg/mL chloramphenicol, and 1 mM IPTG. 1 mL of the cell suspension was added to a 14 mL tube and mixed with 1 mL of a substrate solution comprising 10 mM L-histidine and 10 mM L-cysteine serving as a substrate to start ergothioneine synthesis reaction comprising methylation. The reaction solution was shaken at 200 rpm at 30°C for 48 hours. In order to make sure that methylation reaction could be accelerated by efficient regeneration of methyl group carbon of SAM, a test conducted under the same conditions as above except that glycine serving as a source of methyl group carbon supply was not added was also carried out, and the obtained results were compared with the results of the test conducted under the glycine addition conditions.

The obtained reaction solution was centrifuged at 3000 × g for 5 minutes, and a supernatant was then collected and subjected to LC-MS analysis. The conditions of LC-MS analysis are shown in Table 5.

As a result of measurement, a hercynine concentration and an ergothioneine concentration of the reaction solution were 0.031 mM and 0.054 mM, respectively, under the glycine addition conditions, which were increased by 0.003 mM and 0.015 mM, respectively, as compared with the glycine non-addition conditions. The total concentration of accumulated hercynine and accumulated ergothioneine was 0.084 mM under the glycine addition conditions, which was increased by 0.017 mM as compared with the glycine non-addition conditions.

**[Table 5]**

| | |
|---|---|
| Apparatus | UPLC: Thermo Vanquish UHPLC System Chromeleon 7.2 |
| | MS: Thermo Q Exactive |
| Column | InfinityLab Poroshell 120 HILIC-Z 2.1 mm × 150 mm manufactured by Agilent Technologies, Inc., particle size: 2.7 µm |
| Column temperature | 30°C |
| Mobile phase | (A) 20 mM ammonium formate pH3.0 |
| | (B) Acetonitrile/20 mM ammonium formate pH3.0 |
| Detection | PDA (220-700 nm), MS (ESI, Positive) |
| Flow rate | 0.8 mL/min |
| Elution | 0 min 100% B → 8 min 76% B → 8.5 min 70% B → 8.5 min 100% B → 14.75 min 100% B |

### [Example 39]

The EgtBDE-enhanced strain prepared in Example 33(B) was evaluated for hercynine and ergothioneine production under conditions involving glycine addition at the time of culture as follows.

In this Example, the hercynine and ergothioneine production was evaluated in the same manner as in Example 38 except that after culture in LB medium, M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 10 mM glycine, 0.5% glucose, 50 µg/mL carbenicillin, 50 µg/mL spectinomycin, and 34 µg/mL chloramphenicol was used.

As a result, a hercynine concentration and an ergothioneine concentration of the reaction solution were 0.028 mM and 0.088 mM, respectively, under the glycine addition conditions, which were increased by 0.004 mM and 0.058 mM, respectively, as compared with the glycine non-addition conditions. The total concentration of accumulated hercynine and accumulated ergothioneine was 0.116 mM under the glycine addition conditions, which was increased by 0.062 mM as compared with the glycine non-addition conditions.

### [Example 40]

The EgtBDE, Mtn, LuxS-enhanced strain prepared in Example 34 was evaluated for hercynine and ergothioneine production as follows.

In this Example, the hercynine and ergothioneine production was evaluated in the same manner as in Example 38 except that HMS174(DE3)/pET_P_{T7}-EgtBDE/pCDF_P_{T7}-mtn-luxS prepared in Example 34 was used.

As a result, a hercynine concentration and an ergothioneine concentration of the reaction solution were 0.030 mM and 0.111 mM, respectively, under the glycine addition conditions, which were increased by 0.003 mM and 0.034 mM, respectively, as compared with the glycine non-addition conditions. The total concentration of accumulated hercynine and accumulated ergothioneine was 0.141 mM under the glycine addition conditions, which was increased by 0.037 mM as compared with the glycine non-addition conditions.

### [Example 41]

The EgtBDE, Mtn, LuxS-enhanced strain prepared in Example 34 was evaluated for hercynine and ergothioneine production under conditions involving glycine addition at the time of culture as follows.

In this Example, the hercynine and ergothioneine production was evaluated in the same manner as in Example 38 except that: HMS174(DE3)/pET_P_{T7}-EgtBDE/pCDF_P_{T7}-mtn-luxS prepared in Example 34 was used; and after culture in LB medium, M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 10 mM glycine, 0.5% glucose, 50 µg/mL carbenicillin, 50 µg/mL spectinomycin, and 34 µg/mL chloramphenicol was used.

As a result, a hercynine concentration and an ergothioneine concentration of the reaction solution were 0.083 mM and 0.164 mM, respectively, under the glycine addition conditions, which were increased by 0.051 mM and 0.093 mM, respectively, as compared with the glycine non-addition conditions. The total concentration of accumulated hercynine and accumulated ergothioneine was 0.247 mM under the glycine addition conditions, which was increased by 0.144 mM as compared with the glycine non-addition conditions.

### [Example 42]

The EgtBDE, Mtn, LuxS, Prs, Apt -enhanced strain prepared in Example 35 was evaluated for hercynine and ergothioneine production as follows.

In this Example, the hercynine and ergothioneine production was evaluated in the same manner as in Example 38 except that HMS174(DE3)/pET_P_{T7}-EgtBDE/pCDF_P_{T7}-mtn-luxS-prs-apt prepared in Example 35 was used.

As a result, a hercynine concentration and an ergothioneine concentration of the reaction solution were 0.045 mM and 0.180 mM, respectively, under the glycine addition conditions, which were increased by 0.013 mM and 0.017 mM, respectively, as compared with the glycine non-addition conditions. The total concentration of accumulated hercynine and accumulated ergothioneine was 0.224 mM under the glycine addition conditions, which was increased by 0.029 mM as compared with the glycine non-addition conditions.

### [Example 43]

The EgtBDE, Mtn, LuxS, Prs, Apt -enhanced strain prepared in Example 35 was evaluated for hercynine and ergothioneine production under conditions involving glycine addition at the time of culture as follows.

In this Example, the hercynine and ergothioneine production was evaluated in the same manner as in Example 38 except that: HMS174(DE3)/pET_P_{T7}-EgtBDE/pCDF_P_{T7}-mtn-luxS-prs-apt prepared in Example 35 was used; and after culture in LB medium, M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 10 mM glycine, 0.5% glucose, 50 µg/mL carbenicillin, 50 µg/mL spectinomycin, and 34 µg/mL chloramphenicol was used.

As a result, a hercynine concentration and an ergothioneine concentration of the reaction solution were 0.101 mM and 0.215 mM, respectively, under the glycine addition conditions, which were increased by 0.073 mM and 0.089 mM, respectively, as compared with the glycine non-addition conditions. The total concentration of accumulated hercynine and accumulated ergothioneine was 0.316 mM under the glycine addition conditions, which was increased by 0.162 mM as compared with the glycine non-addition conditions.

### [Example 44]

The EgtBDE, Mtn, LuxS, Prs, Apt, GcvTHP -enhanced strain prepared in Example 36 was evaluated for hercynine and ergothioneine production as follows.

In this Example, the hercynine and ergothioneine production was evaluated in the same manner as in Example 38 except that HMS174(DE3)/pET_P_{T7}-EgtBDE/pCDF_P_{T7}-mtn-luxS-prs-apt/pACYC_P_{T7}-gcvTUP prepared in Example 36 was used.

As a result, a hercynine concentration and an ergothioneine concentration of the reaction solution were 0.071 mM and 0.157 mM, respectively, under the glycine addition conditions, which were increased by 0.048 mM and 0.086 mM, respectively, as compared with the glycine non-addition conditions. The total concentration of accumulated hercynine and accumulated ergothioneine was 0.228 mM under the glycine addition conditions, which was increased by 0.134 mM as compared with the glycine non-addition conditions.

### [Example 45]

The EgtBDE, Mtn, LuxS, Prs, Apt, GcvTHP -enhanced strain prepared in Example 36 was evaluated for hercynine and ergothioneine production under conditions involving glycine addition at the time of culture as follows.

In this Example, the hercynine and ergothioneine production was evaluated in the same manner as in Example 38 except that: HMS174(DE3)/pET_P_{T7}-EgtBDE/pCDF_P_{T7}-mtn-luxS-prs-apt/pACYC_P_{T7}-gcvTUP prepared in Example 36 was used; and after culture in LB medium, M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 10 mM glycine, 0.5% glucose, 50 µg/mL carbenicillin, 50 µg/mL spectinomycin, and 34 µg/mL chloramphenicol was used.

As a result, a hercynine concentration and an ergothioneine concentration of the reaction solution were 0.095 mM and 0.222 mM, respectively, under the glycine addition conditions, which were increased by 0.075 mM and 0.137 mM, respectively, as compared with the glycine non-addition conditions. The total concentration of accumulated hercynine and accumulated ergothioneine was 0.317 mM under the glycine addition conditions, which was increased by 0.212 mM as compared with the glycine non-addition conditions.

### [Example 46]

The EgtBDE, SahH, ADO1, GcvTHP-enhanced strain prepared in Example 37 was evaluated for hercynine and ergothioneine production as follows.

In this Example, the hercynine and ergothioneine production was evaluated in the same manner as in Example 38 except that HMS174(DE3)/pET_P_{T7}-EgtBDE/pCDF_P_{T7}-sahH-ADO1/pACYC_P_{T7}-gcvTHP prepared in Example 37 was used.

As a result, a hercynine concentration and an ergothioneine concentration of the reaction solution were 0.015 mM and 0.018 mM, respectively, under the glycine non-addition conditions, whereas these concentrations were 0.015 mM and 0.036 mM, respectively, under the glycine addition conditions. The total concentration of accumulated hercynine and accumulated ergothioneine was 0.052 mM under the glycine addition conditions, which was increased by 0.019 mM as compared with the glycine non-addition conditions.

### [Example 47]

The EgtBDE, SahH, ADO1, GcvTHP-enhanced strain prepared in Example 37 was evaluated for hercynine and ergothioneine production under conditions involving glycine addition at the time of culture as follows.

In this Example, the hercynine and ergothioneine production was evaluated in the same manner as in Example 38 except that: HMS174(DE3)/pET_P_{T7}-EgtBDE/pCDF_P_{T7}-sahH-ADO1/pACYC_P_{T7}-gcvTUP prepared in Example 37 was used; and after culture in LB medium, M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 10 mM glycine, 0.5% glucose, 50 µg/mL carbenicillin, 50 µg/mL spectinomycin, and 34 µg/mL chloramphenicol was used.

As a result, a hercynine concentration and an ergothioneine concentration of the reaction solution were 0.033 mM and 0.132 mM, respectively, under the glycine addition conditions, which were increased by 0.018 mM and 0.059 mM, respectively, as compared with the glycine non-addition conditions. The total concentration of accumulated hercynine and accumulated ergothioneine was 0.165 mM under the glycine addition conditions, which was increased by 0.077 mM as compared with the glycine non-addition conditions.

The results of Examples 38 to 47 were summarized in Table 6. In order to evaluate methylation reaction efficiency, the total concentration of accumulated hercynine and ergothioneine was calculated and compared. As a result, in these Examples, the total concentrations of accumulated compounds under glycine addition conditions were increased to 1.3 times, 2.1 times, 1.4 times, 2.4 times, 1.1 times, 2.1 times, 2.4 times, 3.0 times, 1.6 times, and 1.9 times, respectively, as compared with those under glycine non-addition conditions. These results demonstrated that performing methylation reaction in the presence of glycine achieves efficient regeneration of SAM and can accelerate the methylation reaction and increase the amount of an arbitrary methyl compound produced.

In Examples 39, 41, 43, 45, and 47, the total concentrations of accumulated compounds under conditions involving glycine addition at the time of methylation reaction were 1.4 times, 1.8 times, 1.4 times, 1.4 times, and 3.2 times, respectively, as compared with those in Examples 38, 40, 42, 44, and 46. These results demonstrated that the preparation of microbial cells by the addition of glycine at the time of culture (before the start of methylation reaction) improves SAM regeneration efficiency and can further accelerate the methylation reaction and increase the amount of an arbitrary methyl compound produced.

Specifically, the present invention was found able to markedly accelerate methylation reaction by culturing cells in the presence of an organic raw material comprising glycine (adding glycine before addition of a methylation target substance, which is before the start of methylation reaction).

In Examples 40 and 41, the total concentrations of accumulated compounds under conditions involving glycine addition at the time of methylation reaction were increased to 1.7 times and 2.1 times, respectively, as compared with those in Examples 38 and 39. These results demonstrated that the enhancement of Mtn and LuxS improves SAM regeneration efficiency and can further accelerate the methylation reaction and increase the amount of an arbitrary methyl compound produced.

In Examples 42 and 43, the total concentrations of accumulated compounds under conditions involving glycine addition at the time of methylation reaction were increased to 1.6 times and 1.3 times, respectively, as compared with those in Examples 40 and 41. These results demonstrated that the enhancement of Prs and Apt improves SAM regeneration efficiency and can further accelerate the methylation reaction and increase the amount of an arbitrary methyl compound produced.

In Example 47, the total concentration of accumulated compounds under conditions involving glycine addition at the time of methylation reaction was increased to 1.4 times that in Example 39. These results demonstrated that the enhancement of Prs, Apt, and GcvTHP improves SAM regeneration efficiency and can further accelerate the methylation reaction and increase the amount of an arbitrary methyl compound produced.

Specifically, the method of the present invention was found able to efficiently regenerate SAM, to accelerate methylation reaction, and to increase the amount of an arbitrary methyl compound produced, by using glycine.

**[Table 6]**

| | Microbial strain | Added component at time of culture | Source of methyl group supply | Total concentration of accumulated hercynine (Her) and ergothioneine (Egt) [mM] | Amount of increase in total concentration of accumulated Her and Egt as compared with glycine non-addition conditions [mM] | Relative value of total concentration of accumulated Her and Egt as compared with glycine non-addition conditions |
|---|---|---|---|---|---|---|
| Example 38 | HMS174 (DE3)/pET_P_{T7}-EgtBDE | - | Glycine | 0.084 | 0.017 | 1.3 |
| Example 39 | | Glycine | Glycine | 0.116 | 0.062 | 2.1 |
| Example 40 | HMS174 (DE3)/pET_P_{T7}-EgtBDE/pCDF_P_{T7}-mtn-luxS | - | Glycine | 0.141 | 0.037 | 1.4 |
| Example 41 | | Glycine | Glycine | 0.247 | 0.144 | 2.4 |
| Example 42 | HMS174 (DE3)/pET_P_{T7}-EgtBDE/pCDF_P_{T7}-mtn-luxS-prs-apt | | Glycine | 0.224 | 0.029 | 1.1 |
| Example 43 | | Glycine | Glycine | 0.316 | 0.162 | 2.1 |
| Example 44 | HMS174 (DE3)/pET_P_{T7}-EgtBDE/pCDF_P_{T7}-mtn-luxS-prs-apt/pACYC_P_{T7}-gcvTHP | | Glycine | 0.228 | 0.134 | 2.4 |
| Example 45 | | Glycine | Glycine | 0.317 | 0.212 | 3.0 |
| Example 46 | HMS174 (DE3)/pET_P_{T7}-EgtBDE/pCDF_P_{T7}-sahH-AD01/pACYC_P_{T7}-gcvTHP | - | Glycine | 0.052 | 0.019 | 1.6 |
| Example 47 | | Glycine | Glycine | 0.165 | 0.077 | 1.9 |

### [Example 48]

The expression of sfmM2 gene and glyA gene was enhanced in *E. coli* HMS174(DE3) strain as follows.

### (A) Construction of GlyA enhancement plasmid

A DNA having a nucleotide sequence (sequence with recognition sequences of restriction enzymes Nde I and Mfe I added to the 5'- and 3'-terminal sides, respectively) comprising *Escherichia* coli-derived glyA gene was synthesized (nucleotide sequence of synthetic DNA: SEQ ID NO: 9). The synthesized DNA was inserted to restriction enzyme sites Nde I and Mfe I of an expression vector pCDF Duet-1 for *E. coli* (Novagen) and linked downstream of T7 promoter. The constructed plasmid was designated as pCDF_P_{T7}-glyA.

### (B) Preparation of SfmM2, GlyA-enhanced strain

*E. coli* HMS174(DE3) strain (Novagen) was transformed with the plasmid pET_P_{T7}-sfmM2 constructed in Example 1(A), the plasmid pCDF_P_{T7}-glyA constructed in Example 48(A), and pACYC Duet-1 (Novagen). The obtained strain was designated as HMS174(DE3)/pET_P_{T7}-sfmM2/pCDF_P_{T7}-glyA.

### [Example 49]

The expression of sfmM2 gene, metF gene, metH gene, metK gene, mtn gene, and luxS gene was enhanced in *E. coli* HMS174(DE3) strain as follows.

### (A) Construction of MetFHK enhancement plasmid

A DNA having a nucleotide sequence (sequence with recognition sequences of restriction enzymes BspHI and EcoRI added to the 5'- and 3'-terminal sides, respectively) comprising *Escherichia* coli-derived metF gene, metH gene, and metK gene (MetF, MetH, and MetK are also collectively referred to as "MetFHK") was synthesized (nucleotide sequence of synthetic DNA: SEQ ID NO: 10). The synthesized DNA was inserted to restriction enzyme sites NcoI and EcoRI of an expression vector pACYC Duet-1 for *E. coli* (Novagen) and linked downstream of T7 promoter. The constructed plasmid was designated as pACYC_P_{T7}-metFHK.

### (B) Construction of MetFHK, Mtn, LuxS enhancement plasmid

The plasmid pCDF_P_{T7}-mtn-luxS constructed in Example 3(A) was treated with restriction enzymes EcoRI and XhoI, separated by agarose gel electrophoresis, then recovered from a gel, and purified to obtain a DNA fragment of the T7 promoter, the mtn gene, and the luxS gene region. The obtained DNA fragment was inserted to the same restriction enzyme sites as above of the plasmid pACYC_P_{T7}-metFHK constructed in Example 49(A). The constructed plasmid was designated as pACYC_P_{T7}-metFHK_P_{T7}-mtn-luxS.

### (C) Preparation of SfmM2, MetFHK, Mtn, LuxS-enhanced strain

*E. coli* HMS174(DE3) strain (Novagen) was transformed with the plasmid pET_P_{T7}-sfmM2 constructed in Example 1(A), the plasmid pACYC_P_{T7}-metFHK_P_{T7}-mtn-luxS constructed in Example 49(B), and pCDF Duet-1 (Novagen). The obtained strain was designated as HMS174(DE3)/pET_P_{T7}-sfmM2/pACYC_P_{T7}-metFHK_P_{T7}-mtn-luxS.

### [Example 50]

The expression of sfmM2 gene, metF gene, metH gene, metK gene, mtn gene, luxS gene, and glyA gene was enhanced in *E. coli* HMS174(DE3) strain as follows.

*E. coli* HMS174(DE3) strain (Novagen) was transformed with the plasmid pET_P_{T7}-sfmM2 constructed in Example 1(A), the plasmid pACYC_P_{T7}-metFHK_P_{T7}-mtn-luxS constructed in Example 49(B), and the plasmid pCDF_P_{T7}-glyA constructed in Example 48(A). The obtained strain was designated as HMS174(DE3)/pET_P_{T7}-sfmM2/pACYC_P_{T7}-metFHK_P_{T7}-mtn-luxS/pCDF_P_{T7}-glyA.

### [Example 51]

The SfmM2-enhanced strain prepared in Example 1(B) was evaluated for 3-methyltyrosine production as follows.

HMS174(DE3)/pET_P_{T7}-sfmM2 prepared in Example 1(B) was cultured overnight in LB medium (16 g/L Bacto-trypton, 10 g/L Yeast extract, 5 g/L NaCl) comprising 50 µg/mL carbenicillin, 50 µg/mL spectinomycin, and 34 µg/mL chloramphenicol. 5 mL of M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 0.5% glucose, 50 µg/mL carbenicillin, 50 µg/mL spectinomycin, and 34 µg/mL chloramphenicol was added to a 50 mL tube, and the culture solution of the LB medium was inoculated at a dilution ratio of 1/50 to the M9 medium, followed by shake culture at 200 rpm at 30°C for 6 hours. IPTG was added thereto at a final concentration of 0.5 mM, followed by further shake culture for 16 hours.

The obtained culture solution was centrifuged at 3000 × g for 5 minutes for harvest and suspended at OD₆₀₀ = 10 in a reaction buffer (100 mM Tris-HCl pH 8.0, 20 mM MgSO₄, 20 mM glucose) comprising 20 mM L-serine, 100 µg/mL carbenicillin, 100 µg/mL spectinomycin, 68 µg/mL chloramphenicol, and 1 mM IPTG. 1 mL of the cell suspension was added to a 14 mL tube and mixed with 1 mL of a 10 mM L-tyrosine solution (dissolved in 0.1 N hydrochloric acid) serving as a substrate to start methylation reaction. The reaction solution was shaken at 200 rpm at 30°C for 24 hours. In order to make sure that methylation reaction could be accelerated by efficient regeneration of methyl group carbon of SAM, a test conducted under the same conditions as above except that L-serine serving as a source of methyl group carbon supply was not added was also carried out, and the obtained results were compared with the results of the test conducted under the L-serine addition conditions.

The obtained reaction solution was subjected to LC-MS analysis in the same manner as in Example 9. As a result of measurement, a 3-methyltyrosine concentration of the reaction solution was 0.221 mM under the L-serine addition conditions, which was increased by 0.093 mM as compared with the L-serine non-addition conditions.

### [Example 52]

The SfmM2-enhanced strain prepared in Example 1(B) was evaluated for 3-methyltyrosine production under conditions involving L-serine addition at the time of culture as follows.

In this Example, the 3-methyltyrosine production was evaluated in the same manner as in Example 51 except that after culture in LB medium, M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 10 mM L-serine, 0.5% glucose, 50 µg/mL carbenicillin, 50 µg/mL spectinomycin, and 34 µg/mL chloramphenicol was used.

As a result, a 3-methyltyrosine concentration of the reaction solution was 0.255 mM under the L-serine addition conditions, which was increased by 0.142 mM as compared with the L-serine non-addition conditions.

### [Example 53]

The SfmM2, GlyA-enhanced strain prepared in Example 48(B) was evaluated for 3-methyltyrosine production as follows.

In this Example, the 3-methyltyrosine production was evaluated in the same manner as in Example 51 except that HMS174(DE3)/pET_P_{T7}-stmM2/pCDF_P_{T7}-glyA prepared in Example 48(B) was used.

As a result, a 3-methyltyrosine concentration of the reaction solution was 0.165 mM under the L-serine addition conditions, which was increased by 0.027 mM as compared with the L-serine non-addition conditions.

### [Example 54]

The SfmM2, GlyA-enhanced strain prepared in Example 48(B) was evaluated for 3-methyltyrosine production under conditions involving L-serine addition at the time of culture as follows.

In this Example, the 3-methyltyrosine production was evaluated in the same manner as in Example 51 except that HMS174(DE3)/pET_P_{T7}-sfmM2/pCDF_P_{T7}-glyA prepared in Example 48(B) was used; and after culture in LB medium, M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 10 mM L-serine, 0.5% glucose, 50 µg/mL carbenicillin, 50 µg/mL spectinomycin, and 34 µg/mL chloramphenicol was used.

As a result, a 3-methyltyrosine concentration of the reaction solution was 0.319 mM under the L-serine addition conditions, which was increased by 0.183 mM as compared with the L-serine non-addition conditions.

### [Example 55]

The SfmM2, MetFHK, Mtn, LuxS-enhanced strain prepared in Example 49(C) was evaluated for 3-methyltyrosine production as follows.

In this Example, the 3-methyltyrosine production was evaluated in the same manner as in Example 51 except that HMS174(DE3)/pET_P_{T7}-sfmM2/pACYC_P_{T7}-metFHK_P_{T7}-mtn-luxS prepared in Example 49(C) was used.

As a result, a 3-methyltyrosine concentration of the reaction solution was 0.243 mM under the L-serine addition conditions, which was increased by 0.131 mM as compared with the L-serine non-addition conditions.

### [Example 56]

The SfmM2, MetFHK, Mtn, LuxS-enhanced strain prepared in Example 49(C) was evaluated for 3-methyltyrosine production under conditions involving L-serine addition at the time of culture as follows.

In this Example, the 3-methyltyrosine production was evaluated in the same manner as in Example 51 except that HMS174(DE3)/pET_P_{T7}-sfmM2/pACYC_P_{T7}-metFHK_P_{T7}-mtn-luxS prepared in Example 49(C) was used; and after culture in LB medium, M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 10 mM L-serine, 0.5% glucose, 50 µg/mL carbenicillin, 50 µg/mL spectinomycin, and 34 µg/mL chloramphenicol was used.

As a result, a 3-methyltyrosine concentration of the reaction solution was 0.543 mM under the L-serine addition conditions, which was increased by 0.324 mM as compared with the L-serine non-addition conditions.

### [Example 57]

The SfmM2, MetFHK, Mtn, LuxS, GlyA-enhanced strain prepared in Example 50 was evaluated for 3-methyltyrosine production as follows.

In this Example, the 3-methyltyrosine production was evaluated in the same manner as in Example 51 except that HMS174(DE3)/pET_P_{T7}-sfmM2/pACYC_P_{T7}-metFHK_P_{T7}-mtn-luxS/pCDF_P_{T7}-glyA prepared in Example 50 was used.

As a result, a 3-methyltyrosine concentration of the reaction solution was 0.240 mM under the L-serine addition conditions, which was increased by 0.047 mM as compared with the L-serine non-addition conditions.

### [Example 58]

The SfmM2, MetFHK, Mtn, LuxS, GlyA-enhanced strain prepared in Example 50 was evaluated for 3-methyltyrosine production under conditions involving L-serine addition at the time of culture as follows.

In this Example, the 3-methyltyrosine production was evaluated in the same manner as in Example 51 except that HMS174(DE3)/pET_P_{T7}-sfmM2/pACYC_P_{T7}-metFHK_P_{T7}-mtn-luxS/pCDF_P_{T7}-glyA prepared in Example 50 was used; and after culture in LB medium, M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 10 mM L-serine, 0.5% glucose, 50 µg/mL carbenicillin, 50 µg/mL spectinomycin, and 34 µg/mL chloramphenicol was used.

As a result, a 3-methyltyrosine concentration of the reaction solution was 0.551 mM under the L-serine addition conditions, which was increased by 0.309 mM as compared with the L-serine non-addition conditions.

The results of Examples 51 to 58 were summarized in Table 7. In these Examples, the concentrations of accumulated 3-methyltyrosine under conditions involving L-serine addition at the time of methylation reaction were drastically increased to 1.7 times, 2.3 times, 1.2 times, 2.2 times, 2.2 times, 2.5 times, 1.2 times, and 2.3 times, respectively, as compared with those under L-serine non-addition conditions. These results demonstrated that performing methylation reaction in the presence of L-serine achieves efficient regeneration of SAM and can accelerate the methylation reaction.

In Examples 52, 54, 56, and 58, the concentrations of accumulated 3-methyltyrosine under conditions involving L-serine addition at the time of methylation reaction were 1.2 times, 1.9 times, 2.2 times, and 2.3 times, respectively, as compared with those in Examples 51, 53, 55, and 57. These results demonstrated that the preparation of microbial cells by the addition of L-serine at the time of culture (before the start of methylation reaction) improves SAM regeneration efficiency and can further accelerate the methylation reaction and increase the amount of an arbitrary methyl compound produced.

Specifically, the present invention was found able to markedly accelerate methylation reaction by culturing cells in the presence of an organic raw material comprising L-serine (adding serine before addition of a methylation target substance which is before the start of methylation reaction).

In Examples 54 and 58, the concentration of accumulated 3-methyltyrosine under conditions involving L-serine addition at the time of methylation reaction was increased by 25% and 1.5%, respectively, as compared with Examples 52 and 56. These results demonstrated that the enhancement of GlyA improves SAM regeneration efficiency and can further accelerate the methylation reaction and increase the amount of an arbitrary methyl compound produced.

**In** Examples 55 and 57, the concentration of accumulated 3-methyltyrosine under conditions involving L-serine addition at the time of methylation reaction was increased by 10% and 45%, respectively, as compared with Examples 51 and 53. **In** Examples 56 and 58, the concentration of accumulated 3-methyltyrosine under conditions involving L-serine addition at the time of methylation reaction was increased by 113% and 73%, respectively, as compared with Examples 52 and 54. These results demonstrated that the enhancement of MetFHK, Mtn, and LuxS improves SAM regeneration efficiency and can further accelerate the methylation reaction and increase the amount of an arbitrary methyl compound produced.

Specifically, the method of the present invention was found able to efficiently regenerate SAM, to accelerate methylation reaction, and to increase the amount of an arbitrary methyl compound produced, by using serine.

**[Table 7]**

| | Microbial strain | Added componen t at time of culture | Sourc e of methy l group supply | Concentration of accumulated 3-methyltyrosin e [mM] | Amount of increase in concentration of accumulated 3-methyltyrosin e as compared with L-serine non-addition conditions [mM] | Relative value of concentration of accumulated 3-methyltyrosin e as compared with L-serine non-addition conditions |
|---|---|---|---|---|---|---|
| Exampl e 51 | HMS174 (DE3)/pET_P_{T7}-sfmM2 | - | L-Serine | 0.221 | 0.093 | 1.7 |
| Exampl e 52 | | L-Serine | L-Serine | 0.255 | 0.142 | 2.3 |
| Exampl e 53 | HMS174 (DE3)/pET_P_{T7}-sfmM2/pCDF_P_{T7}-glyA | - | L-Serine | 0.165 | 0.027 | 1.2 |
| Exampl e 54 | | L-Serine | L-Serine | 0.319 | 0.183 | 2.3 |
| Exampl e 55 | HMS174 (DE3)/pET_P_{T7}-sfmM2/pACYC_P_{T7} -metFHK_P_{T7}-mtn-luxS | - | L-Serine | 0.243 | 0.131 | 2.2 |
| Exampl e 56 | | L-Serine | L-Serine | 0.543 | 0.324 | 2.5 |
| Exampl e 57 | HMS174 (DE3)/pET_P_{T7}-sfmM2/pACYC_P_{T7} -metFHK_P_{T7}-mtn-luxS/pCDF_P_{T7}-glyA | - | L-Serine | 0.240 | 0.047 | 1.2 |
| Exampl e 58 | | L-Serine | L-Serine | 0.551 | 0.309 | 2.3 |

### [Example 59]

The expression of EgtB gene, EgtD gene, and EgtE gene was enhanced in *E. coli* HMS174(DE3) strain as follows.

*E. coli* HMS174(DE3) strain (Novagen) was transformed with the plasmid pET_P_{T7}-EgtBDE constructed in Example 33(A) and pCDF Duet-1 (Novagen). The obtained strain was designated as HMS174(DE3)/pET P_{T7}-EgtBDE.

### [Example 60]

The expression of EgtB gene, EgtD gene, EgtE gene, mtn gene, and luxS gene was enhanced in *E. coli* HMS174(DE3) strain as follows.

*E. coli* HMS174(DE3) strain (Novagen) was transformed with the plasmid pET_P_{T7}-EgtBDE constructed in Example 33(A) and the plasmid pCDF_P_{T7}-mtn-luxS constructed in Example 3(A). The obtained strain was designated as HMS174(DE3)/pET_P_{T7}-EgtBDE/pCDF_P_{T7}-mtn-luxS.

### [Example 61]

The expression of EgtB gene, EgtD gene, EgtE gene, mtn gene, luxS gene, prs gene, and apt gene was enhanced in *E. coli* HMS174(DE3) strain as follows.

*E. coli* HMS174(DE3) strain (Novagen) was transformed with the plasmid pET_P_{T7}-EgtBDE constructed in Example 33(A) and the plasmid pCDF_P_{T7}-mtn-luxS-prs-apt constructed in Example 23(A). The obtained strain was designated as HMS174(DE3)/pET_P_{T7}-EgtBDE/pCDF_P_{T7}-mtn-luxS-prs-apt.

### [Example 62]

The EgtBDE-enhanced strain prepared in Example 59 was evaluated for hercynine and ergothioneine production as follows.

HMS174(DE3)/pET_P_{T7}-EgtBDE prepared in Example 59 was cultured overnight in LB medium (16 g/L Bacto-trypton, 10 g/L Yeast extract, 5 g/L NaCl) comprising 50 µg/mL carbenicillin, and 50 µg/mL spectinomycin. 5 mL of M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 0.5% glucose, 50 µg/mL carbenicillin, and 50 µg/mL spectinomycin was added to a 50 mL tube, and the culture solution of the LB medium was inoculated at a dilution ratio of 1/50 to the M9 medium, followed by shake culture at 200 rpm at 30°C for 6 hours. IPTG was added thereto at a final concentration of 0.5 mM, followed by further shake culture for 16 hours.

The obtained culture solution was centrifuged at 3000 × g for 5 minutes for harvest and suspended at OD₆₀₀ = 4 in a reaction buffer (100 mM Tris-HCl pH 8.0, 20 mM MgSO₄, 20 mM glucose) comprising 20 mM L-serine, 100 µg/mL carbenicillin, 100 µg/mL spectinomycin, and 1 mM IPTG. 1 mL of the cell suspension was added to a 14 mL tube and mixed with 1 mL of a substrate solution comprising 10 mM L-histidine and 10 mM L-cysteine serving as a substrate to start ergothioneine synthesis reaction comprising methylation. The reaction solution was shaken at 200 rpm at 30°C for 48 hours. In order to make sure that methylation reaction could be accelerated by efficient regeneration of methyl group carbon of SAM, a test conducted under the same conditions as above except that L-serine serving as a source of methyl group carbon supply was not added was also carried out, and the obtained results were compared with the results of the test conducted under the L-serine addition conditions.

The obtained reaction solution was subjected to LC-MS analysis in the same manner as in Example 38. As a result of measurement, a hercynine concentration and an ergothioneine concentration of the reaction solution were 0.019 mM and 0.016 mM, respectively, under the L-serine addition conditions, which were increased by 0.008 mM and 0.005 mM, respectively, as compared with the L-serine non-addition conditions. The total concentration of accumulated hercynine and accumulated ergothioneine was 0.035 mM under the L-serine addition conditions, which was increased by 0.012 mM as compared with the L-serine non-addition conditions.

### [Example 63]

The EgtBDE-enhanced strain prepared in Example 59 was evaluated for hercynine and ergothioneine production under conditions involving L-serine addition at the time of culture as follows.

In this Example, the hercynine and ergothioneine production was evaluated in the same manner as in Example 62 except that after culture in LB medium, M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 10 mM L-serine, 0.5% glucose, 50 µg/mL carbenicillin, 50 µg/mL spectinomycin, and 34 µg/mL chloramphenicol was used.

As a result, a hercynine concentration and an ergothioneine concentration of the reaction solution were 0.020 mM and 0.028 mM, respectively, under the L-serine addition conditions, which were increased by 0.007 mM and 0.013 mM, respectively, as compared with the L-serine non-addition conditions. The total concentration of accumulated hercynine and accumulated ergothioneine was 0.049 mM under the L-serine addition conditions, which was increased by 0.020 mM as compared with the L-serine non-addition conditions.

### [Example 64]

The EgtBDE, Mtn, LuxS-enhanced strain prepared in Example 60 was evaluated for hercynine and ergothioneine production as follows.

In this Example, the hercynine and ergothioneine production was evaluated in the same manner as in Example 62 except that HMS174(DE3)/pET_P_{T7}-EgtBDE/pCDF_P_{T7}-mtn-luxS prepared in Example 60 was used.

As a result, a hercynine concentration and an ergothioneine concentration of the reaction solution were 0.230 mM and 0.164 mM, respectively, under the L-serine addition conditions, which were increased by 0.206 mM and 0.110 mM, respectively, as compared with the L-serine non-addition conditions. The total concentration of accumulated hercynine and accumulated ergothioneine was 0.049 mM under the L-serine addition conditions, which was increased by 0.020 mM as compared with the L-serine non-addition conditions.

### [Example 65]

The EgtBDE, Mtn, LuxS-enhanced strain prepared in Example 60 was evaluated for hercynine and ergothioneine production under conditions involving L-serine addition at the time of culture as follows.

In this Example, the hercynine and ergothioneine production was evaluated in the same manner as in Example 62 except that: HMS174(DE3)/pET_P_{T7}-EgtBDE/pCDF_P_{T7}-mtn-luxS prepared in Example 34 was used; and after culture in LB medium, M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 10 mM L-serine, 0.5% glucose, 50 µg/mL carbenicillin, 50 µg/mL spectinomycin, and 34 µg/mL chloramphenicol was used.

As a result, a hercynine concentration and an ergothioneine concentration of the reaction solution were 0.259 mM and 0.166 mM, respectively, under the L-serine addition conditions, which were increased by 0.235 mM and 0.092 mM, respectively, as compared with the L-serine non-addition conditions. The total concentration of accumulated hercynine and accumulated ergothioneine was 0.425 mM under the L-serine addition conditions, which was increased by 0.327 mM as compared with the L-serine non-addition conditions.

### [Example 66]

The EgtBDE, Mtn, LuxS, Prs, Apt-enhanced strain prepared in Example 61 was evaluated for hercynine and ergothioneine production as follows.

In this Example, the hercynine and ergothioneine production was evaluated in the same manner as in Example 62 except that HMS174(DE3)/pET_P_{T7}-EgtBDE/pCDF_P_{T7}-mtn-luxS-prs-apt prepared in Example 61 was used.

As a result, a hercynine concentration and an ergothioneine concentration of the reaction solution were 0.371 mM and 0.168 mM, respectively, under the L-serine addition conditions, which were increased by 0.358 mM and 0.091 mM, respectively, as compared with the L-serine non-addition conditions. The total concentration of accumulated hercynine and accumulated ergothioneine was 0.539 mM under the L-serine addition conditions, which was increased by 0.448 mM as compared with the L-serine non-addition conditions.

### [Example 67]

The EgtBDE, Mtn, LuxS, Prs, Apt-enhanced strain prepared in Example 61 was evaluated for hercynine and ergothioneine production under conditions involving glycine addition at the time of culture as follows.

In this Example, the hercynine and ergothioneine production was evaluated in the same manner as in Example 62 except that HMS174(DE3)/pET_P_{T7}-EgtBDE/pCDF_P_{T7}-mtn-luxS-prs-apt prepared in Example 61 was used; and after culture in LB medium, M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 10 mM L-serine, 0.5% glucose, 50 µg/mL carbenicillin, 50 µg/mL spectinomycin, and 34 µg/mL chloramphenicol was used.

As a result, a hercynine concentration and an ergothioneine concentration of the reaction solution were 0.423 mM and 0.138 mM, respectively, under the L-serine addition conditions, which were increased by 0.413 mM and 0.082 mM, respectively, as compared with the L-serine non-addition conditions. The total concentration of accumulated hercynine and accumulated ergothioneine was 0.561 mM under the L-serine addition conditions, which was increased by 0.495 mM as compared with the L-serine non-addition conditions.

The results of Examples 62 to 67 were summarized in Table 8. In order to evaluate methylation reaction efficiency, the total concentration of accumulated hercynine and ergothioneine was calculated and compared. As a result, in these Examples, the total concentrations of accumulated compounds under glycine addition conditions were increased to 1.5 times, 1.7 times, 5.1 times, 4.3 times, 5.9 times, and 8.5 times, respectively, as compared with those under L-serine non-addition conditions. These results demonstrated that performing methylation reaction in the presence of L-serine achieves efficient regeneration of SAM and can accelerate the methylation reaction and increase the amount of an arbitrary methyl compound produced.

In Examples 63, 65, and 67, the total concentration of accumulated compounds under conditions involving L-serine addition at the time of methylation reaction was increased by 40%, 8%, and 4%, respectively, as compared with Examples 62, 64, and 66. These results demonstrated that the preparation of microbial cells by the addition of L-serine at the time of culture (before the start of methylation reaction) improves SAM regeneration efficiency and can further accelerate the methylation reaction and increase the amount of an arbitrary methyl compound produced.

Specifically, the present invention was found able to markedly accelerate methylation reaction by culturing cells in the presence of an organic raw material comprising serine (adding serine before addition of a methylation target substance which is before the start of methylation reaction).

**In** Examples 64 and 65, the total concentrations of accumulated compounds under conditions involving L-serine addition at the time of methylation reaction were increased to 11.3 times and 8.7 times, respectively, as compared with those in Examples 62 and 63. These results demonstrated that the enhancement of Mtn and LuxS improves SAM regeneration efficiency and can further accelerate the methylation reaction and increase the amount of an arbitrary methyl compound produced.

**In** Examples 66 and 67, the total concentrations of accumulated compounds under conditions involving L-serine addition at the time of methylation reaction were increased to 1.4 times and 1.3 times, respectively, as compared with those in Examples 64 and 65. These results demonstrated that the enhancement of Prs and Apt improves SAM regeneration efficiency and can further accelerate the methylation reaction and increase the amount of an arbitrary methyl compound produced.

Specifically, the method of the present invention was found able to efficiently regenerate SAM, to accelerate methylation reaction, and to increase the amount of an arbitrary methyl compound produced, by using L-serine.

**[Table 8]**

| | Microbial strain | Added component at time of culture | Source of methyl group supply | Total concentration of accumulated hercynine (Her) and ergothioneine (Egt) [mM] | Amount of increase in total concentration of accumulated Her and Egt as compared with L-serine non-addition conditions [mM] | Relative value of total concentration of accumulated Her and Egt as compared with L-serine non-addition conditions |
|---|---|---|---|---|---|---|
| Example 62 | HMS174 (DE3)/pET_P_{T7}-EgtBDE | - | L-Serine | 0.035 | 0.012 | 1.5 |
| Example 63 | | L-Serine | L-Serine | 0.049 | 0.020 | 1.7 |
| Example 64 | HMS174 (DE3)/pET_P_{T7}-EgtBDE/pCDF_P_{T7}-mtn-luxS | - | L-Serine | 0.395 | 0.317 | 5.1 |
| Example 65 | | L-Serine | L-Serine | 0.425 | 0.327 | 4.3 |
| Example 66 | HMS174 (DE3)/pET_P_{T7}-EgtBDE/pCDF_P_{T7}-mtn-luxS-prs-apt | - | L-Serine | 0.539 | 0.448 | 5.9 |
| Example 67 | | L-Serine | L-Serine | 0.561 | 0.495 | 8.5 |

### [Example 68]

The expression of ASMT gene was enhanced in *E*. *coli* HMS174(DE3) strain as follows.

### (A) Construction of ASMT enhancement plasmid

A DNA having a nucleotide sequence (sequence with recognition sequences of restriction enzymes Nco I and Bgl II added to the 5'- and 3'-terminal sides, respectively) comprising *Homo sapiens-derived* ASMT gene encoding acetylserotonin O-methyltransferase was synthesized (nucleotide sequence of synthetic DNA: SEQ ID NO: 11). The nucleotide sequence of the ASMT gene was designed as a codon-optimized sequence for efficient expression in *E. coli* with alanine at position 258 mutated to glutamic acid. The synthesized DNA was inserted to restriction enzyme sites Nco I and Bgl II of an expression vector pET Duet-1 for *E. coli* (Novagen) and linked downstream of T7 promoter. The constructed plasmid was designated as pET_P_{T7}-ASMT.

### (B) Preparation of ASMT-enhanced strain

*E. coli* HMS174(DE3) strain (Novagen) was transformed with the plasmid pET_P_{T7}-ASMT constructed in Example 68(A) and pCDFDuet-1 (Novagen). The obtained strain was designated as HMS174(DE3)/pET_P_{T7}-ASMT.

### [Example 69]

The expression of ASMT gene, mtn gene, and luxS gene was enhanced in *E. coli* HMS174(DE3) strain as follows.

*E. coli* HMS174(DE3) strain (Novagen) was transformed with the plasmid pET_P_{T7}-ASMT constructed in Example 68(A) and the plasmid pCDF_P_{T7}-mtn-luxS constructed in Example 3(A). The obtained strain was designated as HMS174(DE3)/pET_P_{T7}-ASMT/pCDF_P_{T7}-mtn-luxS.

### [Example 70]

The expression of ASMT gene, mtn gene, luxS gene, Prs gene, and Apt gene was enhanced in *E. coli* HMS174(DE3) strain as follows.

*E. coli* HMS174(DE3) strain (Novagen) was transformed with the plasmid pET_P_{T7}-ASMT constructed in Example 68(A) and the plasmid pCDF_P_{T7}-mtn-luxS-prs-apt constructed in Example 23(A). The obtained strain was designated as HMS174(DE3)/pET_P_{T7}-ASMT/pCDF_P_{T7}-mtn-luxS-prs-apt.

### [Example 71]

The expression of ASMT gene, glyA gene, mtn gene, and luxS gene was enhanced in *E. coli* HMS174(DE3) strain as follows.

### (A) Construction of GlyA, Mtn, LuxS enhancement plasmid

A DNA having a nucleotide sequence (sequence with recognition sequences of restriction enzymes Mfe I and Kpn I added to the 5'- and 3'-terminal sides, respectively) comprising *Escherichia* coli-derived mtn gene and luxS gene was synthesized (nucleotide sequence of synthetic DNA: SEQ ID NO: 12). The synthesized DNA was inserted to restriction enzyme sites Mfe I and Kpn I of the plasmid pCDF_P_{T7}-glyA constructed in Example 48(A), and linked downstream of the glyA gene. The constructed plasmid was designated as pCDF_P_{T7}-glyA-mtn-luxS.

### (B) Preparation of ASMT, GlyA, Mtn, LuxS-enhanced strain

*E. coli* HMS174(DE3) strain (Novagen) was transformed with the plasmid pET_P_{T7}-ASMT constructed in Example 68(A) and the plasmid pCDF_P_{T7}-glyA-mtn-luxS constructed in Example 71(A). The obtained strain was designated as HMS174(DE3)/pET_P_{T7}-ASMT/pCDF_P_{T7}-glyA-mtn-luxS.

### [Example 72]

The expression of ASMT gene, glyA gene, mtn gene, luxS gene, Prs gene, and Apt gene was enhanced *in E. coli* HMS174(DE3) strain as follows.

### (A) Construction of GlyA, Mtn, LuxS, Prs, Apt enhancement plasmid

A DNA having a nucleotide sequence (sequence with recognition sequences of restriction enzymes Kpn I and Avr II added to the 5'- and 3'-terminal sides, respectively) comprising *Escherichia* coli-derived prs gene and apt gene was synthesized (nucleotide sequence of synthetic DNA: SEQ ID NO: 13). The synthesized DNA was inserted to restriction enzyme sites Kpn I and Avr II of the plasmid pCDF_P_{T7}-glyA-mtn-luxS constructed in Example 71(A), and linked downstream of the glyA gene. The constructed plasmid was designated as pCDF_P_{T7}-glyA-mtn-luxS-prs-apt.

### (B) Preparation of ASMT, GlyA, Mtn, LuxS, Prs, Apt-enhanced strain

*E. coli* HMS174(DE3) strain (Novagen) was transformed with the plasmid pET_P_{T7}-ASMT constructed in Example 68(A) and the plasmid pCDF_P_{T7}-glyA-mtn-luxS-prs-apt constructed in Example 72(A). The obtained strain was designated as HMS174(DE3)/pET_P_{T7}-ASMT/pCDF_P_{T7}-glyA-mtn-luxS-prs-apt.

### [Example 73]

The ASMT-enhanced strain prepared in Example 68(B) was evaluated for melatonin production as follows.

HMS174(DE3)/pET_P_{T7}-ASMT prepared in Example 68(B) was cultured overnight in LB medium (16 g/L Bacto-trypton, 10 g/L Yeast extract, 5 g/L NaCl) comprising 50 µg/mL carbenicillin and 50 µg/mL spectinomycin. 5 mL of M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 10 mM L-serine, 0.5% glucose, 50 µg/mL carbenicillin, and 50 µg/mL spectinomycin was added to a 50 mL tube, and the culture solution of the LB medium was inoculated at a dilution ratio of 1/50 to the M9 medium, followed by shake culture at 200 rpm at 30°C for 6 hours. IPTG was added thereto at a final concentration of 0.5 mM, followed by further shake culture for 16 hours.

The obtained culture solution was centrifuged at 3000 × g for 5 minutes for harvest and suspended at OD₆₀₀ = 4 in a reaction buffer (100 mM Tris-HCl pH 8.0, 20 mM MgSO₄, 20 mM glucose) comprising 20 mM L-serine, 100 µg/mL carbenicillin, 100 µg/mL spectinomycin, and 1 mM IPTG. 1 mL of the cell suspension was added to a 14 mL tube and mixed with 1 mL of a substrate solution comprising 50 mM N-acetylserotonin serving as a substrate to start melatonin synthesis reaction comprising methylation. The reaction solution was shaken at 200 rpm at 30°C for 48 hours.

In order to make sure that methylation reaction could be accelerated by efficient regeneration of methyl group carbon of SAM, a test conducted under the same conditions as above except that L-serine serving as a source of methyl group carbon supply was not added was also carried out, and the obtained results were compared with the results of the test conducted under the L-serine addition conditions.

100 µL of the obtained reaction solution was mixed with 100 µL of methanol, and a supernatant was collected after centrifugation at 3000 × g for 5 minutes and subjected to LC-MS analysis. The conditions of LC-MS analysis are shown in Table 9.

As a result of measurement, a melatonin concentration of the reaction solution was 0.094 mM under the L-serine addition conditions, which was increased by 0.003 mM as compared with the L-serine non-addition conditions.

**[Table 9]**

| | |
|---|---|
| Apparatus | UPLC: Thermo Vanquish UHPLC System Chromeleon 7.2 MS: Thermo Q Exactive |
| Column | ACQUITY UPLC BEH C18 2.1 mm × 50 mm manufactured by Waters Corp., particle size: 1.7 µm |
| Column temperature | 42°C |
| Mobile phase | (A) 0.1% formic acid (B) Methanol |
| Detection | PDA (220-700 nm), MS (ESI, Positive) |
| Flow rate | 0-8 min: 0.2 ml/min → 8-9.5 min: 0.5 ml/min → 9.5-10 min: 0.2 ml/min |
| Elution | 0 min 5% B → 5 min 40% B → 7 min 100% B → 8 min 100% B → 8 min 5% B → 9.5 min 5% B → 10 min 5% B |

### [Example 74]

The ASMT, Mtn, LuxS-enhanced strain prepared in Example 69 was evaluated for melatonin production as follows.

In this Example, the melatonin production was evaluated in the same manner as in Example 73 except that HMS174(DE3)/pET_P_{T7}-ASMT/pCDF_P_{T7}-mtn-luxS prepared in Example 69 was used.

As a result, a melatonin concentration of the reaction solution was 0.220 mM under the L-serine addition conditions, which was increased by 0.012 mM as compared with the L-serine non-addition conditions.

### [Example 75]

The ASMT, Mtn, LuxS, Prs, Apt-enhanced strain prepared in Example 70 was evaluated for melatonin production as follows.

In this Example, the melatonin production was evaluated in the same manner as in Example 73 except that HMS174(DE3)/pET_P_{T7}-ASMT/pCDF_P_{T7}-mtn-luxS-prs-apt prepared in Example 70 was used.

As a result, a melatonin concentration of the reaction solution was 0.248 mM under the L-serine addition conditions, which was increased by 0.016 mM as compared with the L-serine non-addition conditions.

### [Example 76]

The ASMT, GlyA, Mtn, LuxS-enhanced strain prepared in Example 71(B) was evaluated for melatonin production as follows.

In this Example, the melatonin production was evaluated in the same manner as in Example 73 except that HMS174(DE3)/pET_P_{T7}-ASMT/pCDF_P_{T7}-glyA-mtn-luxS prepared in Example 71(B) was used.

As a result, a melatonin concentration of the reaction solution was 0.295 mM under the L-serine addition conditions, which was increased by 0.004 mM as compared with the L-serine non-addition conditions.

### [Example 77]

The ASMT, GlyA, Mtn, LuxS, Prs, Apt-enhanced strain prepared in Example 72(B) was evaluated for melatonin production as follows.

In this Example, the melatonin production was evaluated in the same manner as in Example 73 except that HMS174(DE3)/pET_P_{T7}-ASMT/pCDF_P_{T7}-glyA-mtn-luxS-prs-apt prepared in Example 72(B) was used.

As a result, a melatonin concentration of the reaction solution was 0.301 mM under the L-serine addition conditions, which was increased by 0.029 mM as compared with the L-serine non-addition conditions.

The results of Examples 73 to 77 were summarized in Table 10. In these Examples, the concentration of accumulated melatonin under L-serine addition conditions was increased by 3%, 6%, 7%, 16%, and 11%, respectively, as compared with L-serine non-addition conditions. These results demonstrated that performing methylation reaction in the presence of L-serine achieves efficient regeneration of SAM and can accelerate the methylation reaction and increase the amount of an arbitrary methyl compound produced.

In Example 74, the concentration of accumulated melatonin under L-serine addition conditions was increased to 2.3 times the results of Example 73. These results demonstrated that the enhancement of the expression of Mtn and LuxS improves SAM regeneration efficiency and can further accelerate the methylation reaction.

In Examples 75 and 77, the concentration of accumulated melatonin under L-serine addition conditions was increased by 13% and 2%, respectively, as compared with the results of Examples 74 and 76. These results demonstrated that the enhancement of the expression of Mtn and LuxS, as well as the expression of Prs and Apt, improves SAM regeneration efficiency and can further accelerate the methylation reaction.

In Examples 76 and 77, the concentration of accumulated melatonin under L-serine addition conditions was increased by 34% and 21%, respectively, as compared with the results of Examples 74 and 75. These results demonstrated that the enhancement of the expression of Mtn, LuxS, Prs, and Apt, as well as the expression of GlyA, improves SAM regeneration efficiency and can further accelerate the methylation reaction.

Specifically, the method of the present invention was found able to efficiently regenerate SAM, to accelerate methylation reaction, and to increase the amount of an arbitrary methyl compound produced, by using L-serine.

**[Table 10]**

| | Microbial strain | Added component at time of culture | Source of methyl group supply | Concentration of accumulated melatonin [mM] | Amount of increase in concentration of accumulated melatonin as compared with L-serine non-addition conditions [mM] | Rate of increase in concentration of accumulated melatonin as compared with L-serine non-addition conditions (%) |
|---|---|---|---|---|---|---|
| Example 73 | HMS174 (DE3)/pET_PT7-ASMT | L-Serine | L-Serine | 0.094 | 0.003 | 3 |
| Example 74 | HMS174 (DE3)/pET_PT7-ASMT/pCDF_PT7-mtn-luxS | L-Serine | L-Serine | 0.220 | 0.012 | 6 |
| Example 75 | HMS174 (DE3)/pET_PT7-ASMT/pCDF _PT7-mtn-luxS-prs-apt | L-Serine | L-Serine | 0.248 | 0.016 | 7 |
| Example 76 | HMS174 (DE3)/pET_PT7-ASMT/pCDF _PT7-glyA-mtn-luxS | L-Serine | L-Serine | 0.295 | 0.004 | 16 |
| Example 77 | HMS174 (DE3)/pET_PT7-ASMT/pCDF_PT7-glyA-mtn-luxS-prsapt | L-Serine | L-Serine | 0.301 | 0.029 | 11 |

### [Example 78]

The SfmM2, GlyA-enhanced strain prepared in Example 48(B) was evaluated for 3-methyltyrosine production as follows.

HMS174(DE3)/pET_P_{T7}-sfmM2/pCDF_P_{T7}-glyA prepared in Example 48(B) was cultured overnight in LB medium (16 g/L Bacto-trypton, 10 g/L Yeast extract, 5 g/L NaCl) comprising 50 µg/mL carbenicillin, 50 µg/mL spectinomycin, and 34 µg/mL chloramphenicol. 5 mL of M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 0.5% glucose, 50 µg/mL carbenicillin, 50 µg/mL spectinomycin, and 34 µg/mL chloramphenicol was added to a 50 mL tube, and the culture solution of the LB medium was inoculated at a dilution ratio of 1/50 to the M9 medium, followed by shake culture at 200 rpm at 30°C for 6 hours. IPTG was added thereto at a final concentration of 0.5 mM, followed by further shake culture for 16 hours.

The obtained culture solution was centrifuged at 3000 × g for 5 minutes for harvest and suspended at OD₆₀₀ = 10 in a reaction buffer (100 mM Tris-HCl pH 8.0, 20 mM MgSO₄, 20 mM glucose) comprising 100 µg/mL carbenicillin, 100 µg/mL spectinomycin, 68 µg/mL chloramphenicol, and 1 mM IPTG. 1 mL of the cell suspension was added to a 14 mL tube and mixed with 1 mL of a 10 mM L-tyrosine solution (dissolved in 0.1 N hydrochloric acid) serving as a substrate to start methylation reaction. The reaction solution was shaken at 200 rpm at 30°C for 6 hours.

The obtained reaction solution was subjected to LC-MS analysis in the same manner as in Example 9. As a result of measurement, a 3-methyltyrosine concentration of the reaction solution was 0.075 mM.

### [Example 79]

The SfmM2, MetFHK, Mtn, LuxS, GlyA-enhanced strain prepared in Example 50 was evaluated for 3-methyltyrosine production as follows.

In this Example, the 3-methyltyrosine production was evaluated in the same manner as in Example 78 except that HMS 174(DE3)/pET_P_{T7}-sfmM2/pACYC_P_{T7}-metFHK_P_{T7}-mtn-luxS/pCDF_P_{T7}-glyA prepared in Example 50 was used.

As a result, a 3-methyltyrosine concentration of the reaction solution was 0.170 mM.

### [Comparative Example 1]

The SfmM2-enhanced strain prepared in Example 1 was evaluated for 3-methyltyrosine production as follows.

In this Comparative Example, the 3-methyltyrosine production was evaluated in the same manner as in Example 78 except that HMS 174(DE3)/pET_P_{T7}-sfmM2 prepared in Example 1 was used.

As a result, a 3-methyltyrosine concentration of the reaction solution was 0.036 mM.

### [Comparative Example 2]

The SfmM2, MetFHK, Mtn, LuxS-enhanced strain prepared in Example 49(C) was evaluated for 3-methyltyrosine production as follows.

In this Comparative Example, the 3-methyltyrosine production was evaluated in the same manner as in Example 78 except that HMS174(DE3)/pET_P_{T7}-sfmM2/pACYC_P_{T7}-metFHK_P_{T7}-mtn-luxS prepared in Example 49(C) was used.

As a result, a 3-methyltyrosine concentration of the reaction solution was 0.063 mM.

### [Comparative Example 3]

The SfmM2, GcvTHP-enhanced strain prepared in Example 2(B) was evaluated for 3-methyltyrosine production as follows.

In this Comparative Example, the 3-methyltyrosine production was evaluated in the same manner as in Example 78 except that HMS174(DE3)/pET_P_{T7}-sfmM2/pACYC_P_{T7}-gcvTHP prepared in Example 2(B) was used.

As a result, a 3-methyltyrosine concentration of the reaction solution was 0.021 mM.

### [Comparative Example 4]

The SfmM2, Mtn, LuxS-enhanced strain prepared in Example 5 was evaluated for 3-methyltyrosine production as follows.

In this Comparative Example, the 3-methyltyrosine production was evaluated in the same manner as in Example 78 except that HMS174(DE3)/pET_P_{T7}-sfmM2/pCDF_P_{T7}-mtn-luxS prepared in Example 5 was used.

As a result, a 3-methyltyrosine concentration of the reaction solution was 0.029 mM.

### [Comparative Example 5]

The SfmM2, Mtn, LuxS, GcvTHP-enhanced strain prepared in Example 6 was evaluated for 3-methyltyrosine production as follows.

In this Comparative Example, the 3-methyltyrosine production was evaluated in the same manner as in Example 78 except that HMS174(DE3)/pET_P_{T7}-sfmM2/pCDF_P_{T7}-mtn-luxS/pACYC_P_{T7}-gcvTHP prepared in Example 6 was used.

As a result, a 3-methyltyrosine concentration of the reaction solution was 0.020 mM.

The results of Examples 78 and 79 and Comparative Examples 1 to 5 were summarized in Table 11. In Examples 78 and 79, the concentrations of accumulated 3-methyltyrosine were drastically increased to 2.1 times and 2.7 times, respectively, as compared with those in Comparative Examples 1 and 2. These results demonstrated that the enhancement of GlyA achieves efficient regeneration of SAM using glucose as a source of methyl group carbon supply and can accelerate the methylation reaction and increase the amount of an arbitrary methyl compound produced. It is considered that methyl group carbon of SAM is regenerated from glucose through L-serine.

In Example 79, the concentration of accumulated 3-methyltyrosine was drastically increased to 2.3 times as that in Example 78. This demonstrated that the enhancement of MetFHK, Mtn, and LuxS improves SAM regeneration efficiency and can further accelerate the methylation reaction and increase the amount of an arbitrary methyl compound produced.

Specifically, the present invention was found able to efficiently regenerate SAM from an organic raw material such as glucose, to accelerate methylation reaction, and to increase the amount of an arbitrary methyl compound produced, by using the engineered cell of the present invention.

On the other hand, Comparative Examples 3 and 5 compared with Comparative Examples 1 and 4, respectively, exhibited no increase in accumulation of 3-methyltyrosine. Although a metabolic pathway through which methyl group carbon of SAM is regenerated via glycine produced from glucose may be possible, the methylation reaction could not be accelerated by the enhancement of GcvTHP, suggesting that the enhancement of GlyA is important for using a general organic raw material such as glucose as a source of methyl group carbon supply.

**[Table 11]**

| | Microbial strain | Source of methyl group supply | Concentration of accumulated 3-methyltyrosine [mM] |
|---|---|---|---|
| Example 78 | HMS174 (DE3)/pET_P_{T7}-sfmM2/pCDF_P_{T7}-glyA | Glucose | 0.075 |
| Example 79 | HMS174 (DE3)/pET_P_{T7}-sfmM2/pACYC_P_{T7}-metFHK_P_{T7}-mtn-luxs/pCDF_P_{T7}-glyA | Glucose | 0.170 |
| Comparative Example 1 | HMS174 (DE3)/pET_P_{T7}-stmM2 | Glucose | 0.036 |
| Comparative Example 2 | HMS174 (DE3)/pET_P_{T7}-stmM2/pACYC_P_{T7}-metFHK_P_{T7}-mtn-luxS | Glucose | 0.063 |
| Comparative Example 3 | HMS174 (DE3)/pET_P_{T7}-sfmM2/pACYC_P_{T7}-gcvTHP | Glucose | 0.021 |
| Comparative Example 4 | HMS174(DE3)/pCDF_P_{T7}-mtn-luxS | Glucose | 0.029 |
| Comparative Example 5 | HMS174 (DE3)/pET_P_{T7}-sfmM2/pCDF_P_{T7}-mtn-luxS/pACYC_P_{T7}-gcvTHP | Glucose | 0.020 |

### [Example 80]

The ASMT, GlyA, Mtn, LuxS-enhanced strain prepared in Example 71(B) was evaluated for melatonin production as follows.

HMS174(DE3)/pET_P_{T7}-ASMT/pCDF_P_{T7}-glyA-mtn-luxS prepared in Example 71(B) was cultured overnight in LB medium (16 g/L Bacto-trypton, 10 g/L Yeast extract, 5 g/L NaCl) comprising 50 µg/mL carbenicillin and 50 µg/mL spectinomycin. 5 mL of M9 medium (6 g/L Na₂HPO₄, 3 g/L KH₂PO₄, 0.5 g/L NaCl, 1 g/L NH₄Cl, 2 mM MgSO₄·7H₂O, 0.1 mM CaCl₂·2H₂O, 10 mg/L thiamine hydrochloride) comprising 0.5% glucose, 50 µg/mL carbenicillin, and 50 µg/mL spectinomycin was added to a 50 mL tube, and the culture solution of the LB medium was inoculated at a dilution ratio of 1/50 to the M9 medium, followed by shake culture at 200 rpm at 30°C for 6 hours. IPTG was added thereto at a final concentration of 0.5 mM, followed by further shake culture for 16 hours.

The obtained culture solution was centrifuged at 3000 × g for 5 minutes for harvest and suspended at OD₆₀₀ = 4 in a reaction buffer (100 mM Tris-HCl pH 8.0, 20 mM MgSO₄, 20 mM glucose) comprising 100 µg/mL carbenicillin, 100 µg/mL spectinomycin, and 1 mM IPTG. 1 mL of the cell suspension was added to a 14 mL tube and mixed with 1 mL of a substrate solution comprising 50 mM N-acetylserotonin serving as a substrate to start melatonin synthesis reaction comprising methylation. The reaction solution was shaken at 200 rpm at 30°C for 24 hours.

In order to make sure that methylation reaction could be accelerated by efficient regeneration of methyl group carbon of SAM, a test conducted under the same conditions as above except that L-serine serving as a source of methyl group carbon supply was not added was also carried out, and the obtained results were compared with the results of the test conducted under the L-serine addition conditions.

The obtained reaction solution was subjected to LC-MS analysis in the same manner as in Example 73. As a result of measurement, a melatonin concentration of the reaction solution was 0.248 mM.

### [Example 81]

The ASMT, GlyA, Mtn, LuxS, Prs, Apt-enhanced strain prepared in Example 72(B) was evaluated for melatonin production as follows.

In this Example, the melatonin production was evaluated in the same manner as in Example 80 except that HMS174(DE3)/pET_P₁₇-ASMT/pCDF_P₁₇-glyA-mtn-luxS-prs-apt prepared in Example 72(B) was used.

As a result, a melatonin concentration of the reaction solution was 0.281 mM.

### [Comparative Example 6]

The ASMT-enhanced strain prepared in Example 68(B) was evaluated for melatonin production as follows.

In this Example, the melatonin production was evaluated in the same manner as in Example 80 except that HMS174(DE3)/pET_P_{T7}-ASMT prepared in Example 68(B) was used.

As a result, a melatonin concentration of the reaction solution was 0.075 mM.

### [Comparative Example 7]

The ASMT, Mtn, LuxS-enhanced strain prepared in Example 69 was evaluated for melatonin production as follows.

In this Example, the melatonin production was evaluated in the same manner as in Example 80 except that HMS174(DE3)/pET_P_{T7}-ASMT/pCDF_P_{T7}-glyA-mtn-luxS prepared in Example 69 was used.

As a result, a melatonin concentration of the reaction solution was 0.189 mM.

### [Comparative Example 8]

The ASMT, Mtn, LuxS, Prs, Apt-enhanced strain prepared in Example 70 was evaluated for melatonin production as follows.

In this Example, the melatonin production was evaluated in the same manner as in Example 80 except that HMS174(DE3)/pET_P₁₇-ASMT/pCDF_P₁₇-glyA-mtn-luxS-prs-apt prepared in Example 70 was used.

As a result, a melatonin concentration of the reaction solution was 0.263 mM.

The results of Examples 80 and 81 and Comparative Examples 6 to 8 were summarized in Table 12. In Examples 80 and 81, the concentration of accumulated melatonin was increased by 31% and 7%, respectively, as compared with Comparative Examples 7 and 8. These results demonstrated that the enhancement of GlyA achieves efficient regeneration of SAM using glucose as a source of methyl group carbon supply and can accelerate the methylation reaction and increase the amount of an arbitrary methyl compound produced. It is considered that methyl group carbon of SAM is regenerated from glucose through L-serine.

In Example 81, the concentration of accumulated melatonin was increased by 13% as compared with Example 80. This demonstrated that the enhancement of the expression of Mtn and LuxS, as well as the expression of Prs and Apt, improves SAM regeneration efficiency and can further accelerate the methylation reaction and increase the amount of an arbitrary methyl compound produced.

Specifically, the present invention was found able to efficiently regenerate SAM from an organic raw material such as glucose, to accelerate methylation reaction, and to increase the amount of an arbitrary methyl compound produced, by using the engineered cell of the present invention.

**[Table 12A]**

| | Microbial strain | Source of methyl group supply | Concentration of accumulated melatonin [mM] |
|---|---|---|---|
| Example 80 | HMS174 (DE3)/pET_PT7-ASMT/pCDF_PT7-glyA-mtn-luxS | Glucose | 0.248 |
| Comparative Example 6 | HMS174 (DE3)/pET_PT7-ASMT | Glucose | 0.075 |
| Comparative Example 7 | HMS174 (DE3)/pET_PT7-ASMT/pCDF_PT7-mtn-luxS | Glucose | 0.189 |

**[Table 12B]**

| | Microbial strain | Source of methyl group supply | Concentration of accumulated melatonin [mM] |
|---|---|---|---|
| Example 81 | HMS174 (DE3)/pET_PT7-ASMT/pCDF_PT7-glyA-mtn-luxS-prs-apt | Glucose | 0.281 |
| Comparative Example 6 | HMS174 (DE3)/pET_PT7-ASMT | Glucose | 0.075 |
| Comparative Example 8 | HMS174 (DE3)/pET_PT7-ASMT/pCDF_PT7-mtn-luxS-prs-apt | Glucose | 0.263 |

### [Industrial Applicability]

The present invention can produce a methyl compound by accelerating the methylation reaction of a compound.

### [Free Text of Sequence Listing]

SEQ ID NOs: 1 to 13: synthetic DNA

## Claims

1. A method for producing a methyl compound, comprising the step of performing methylation reaction in the presence of glycine or serine by using a cell engineered so as to enhance an activity or expression of S-adenosylmethionine-dependent methyltransferase.

2. The method according to claim 1, wherein the cell is a cell further engineered so as to enhance an activity or expression of serine hydroxymethyltransferase (GlyA).

3. A method for producing a methyl compound, comprising the step of performing methylation reaction using a cell engineered so as to enhance an activity or expression of serine hydroxymethyltransferase (GlyA) and S-adenosylmethionine-dependent methyltransferase.

4. The method according to claim 3, wherein the cell is a cell further engineered so as to enhance an activity or expression of at least one or more enzymes selected from the group consisting of D-3-phosphoglycerate dehydrogenase (SerA), phosphoserine aminotransferase (SerC), and phosphoserine phosphatase (SerB).

5. The method according to claim 1 or 3, wherein the cell is a cell further engineered so as to enhance an activity or expression of at least one or more enzymes selected from the group consisting of S-adenosylhomocysteine nucleosidase (Mtn), S-ribosylhomocysteine lyase (LuxS), and S-adenosylhomocysteine hydrolase (SahH).

6. The method according to claim 1 or 3, wherein the cell is a cell further engineered so as to enhance an activity or expression of at least one or more enzymes selected from the group consisting of ribose-phosphate diphosphokinase (Prs), adenine phosphoribosyltransferase (Apt), and adenosine kinase (ADK).

7. The method according to claim 1 or 3, wherein the cell is a cell further engineered so as to enhance an activity or expression of at least one or more enzymes selected from the group consisting of 5,10-methylene-tetrahydrofolate reductase (MetF), methionine synthase (MetH or MetE), and methionine adenosyltransferase (MetK).

8. The method according to claim 1 or 3, wherein the cell is a cell further engineered so as to enhance an activity or expression of at least one or more enzymes selected from the group consisting of aminomethyltransferase (GcvT), octanoyl-[GcvH]:protein N-octanoyltransferase (GcvH), and glycine dehydrogenase (GcvP).

9. The method according to claim 1 or 3, wherein the cell is at least one selected from the group consisting of an animal cell, an insect cell, a plant cell, and a microorganism.

10. The method according to claim 9, wherein the cell is a microorganism.

11. The method according to claim 10, wherein the microorganism is at least one selected from the group consisting of *E. coli,* a coryneform bacterium, a bacterium of the genus *Bacillus,* a bacterium of the genus *Pantoea,* a bacterium of the genus *Enterobacter,* a bacterium of the genus *Pseudomonas,* a bacterium of the genus *Streptomyces,* a bacterium of the genus *Actinomyces,* a filamentous fungus, and a yeast.

12. The method according to claim 1 or 3, further comprising the step of allowing an organic raw material to act on the cell.

13. The method according to claim 12, wherein the organic raw material is an organic compound having 3 or more carbon atoms.

14. The method according to claim 12, wherein the organic raw material comprises one selected from the group consisting of a saccharide, an alcohol and an organic acid.

15. The method according to claim 1, wherein the cell is a cell cultured in the presence of an organic raw material containing glycine or serine.

16. The method according to claim 1 or 3, further comprising the step of recovering a methyl compound produced through the methylation reaction.

17. The method according to claim 1 or 3, further comprising the step of purifying a methyl compound produced through the methylation reaction.

18. The method according to claim 12, further comprising the step of recovering a methyl compound produced by allowing the organic raw material to act.

19. The method according to claim 12, further comprising the step of purifying a methyl compound produced by allowing the organic raw material to act.

20. The method according to claim 1 or 3, wherein the methyl compound is a derivative of an aromatic amino acid.

21. A cell engineered so as to enhance an activity or expression of serine hydroxymethyltransferase (GlyA) and S-adenosylmethionine-dependent methyltransferase.

22. The cell according to claim 21, further engineered so as to enhance an activity or expression of at least one or more enzymes selected from the group consisting of S-adenosylhomocysteine nucleosidase (Mtn), S-ribosylhomocysteine lyase (LuxS), and S-adenosylhomocysteine hydrolase (SahH).

23. The cell according to claim 21, further engineered so as to enhance the activity or expression of at least one or more enzymes selected from the group consisting of ribose-phosphate diphosphokinase (Prs), adenine phosphoribosyltransferase (Apt), and adenosine kinase (ADK).

24. The cell according to claim 21, further engineered so as to enhance the activity or expression of at least one or more enzymes selected from the group consisting of 5,10-methylene-tetrahydrofolate reductase (MetF), methionine synthase (MetH or MetE), and methionine adenosyltransferase (metK).

25. The cell according to claim 21, further engineered so as to enhance the activity or expression of at least one or more enzymes selected from the group consisting of aminomethyltransferase (GcvT), octanoyl-[GcvH]:protein N-octanoyltransferase (GcvH), and glycine dehydrogenase (GcvP).

26. The cell according to any one of claims 21 to 25, further engineered so as to enhance the activity or expression of at least one or more enzymes selected from the group consisting of D-3-phosphoglycerate dehydrogenase (SerA), phosphoserine aminotransferase (SerC), and phosphoserine phosphatase (SerB).

27. The cell according to claim 21, wherein the cell is at least one selected from the group consisting of an animal cell, an insect cell, a plant cell, and a microorganism.

28. The cell according to claim 27, wherein the cell is a microorganism.

29. The cell according to claim 28, wherein the microorganism is at least one selected from the group consisting of *E. coli,* a coryneform bacterium, a bacterium of the genus *Bacillus, a* bacterium of the genus *Pantoea,* a bacterium of the genus *Enterobacter,* a bacterium of the genus *Pseudomonas,* a bacterium of the genus *Streptomyces,* a bacterium of the genus *Actinomyces,* a filamentous fungus, and a yeast.

30. A method for producing an organic compound, comprising the step of producing an organic compound via the methyl compound obtained by the method according to claim 1 or 3 as an intermediate.
